# EUROPÄISCHE PATENTSCHRIFT

(11) **EP 0 693 080 B1**
(45) Veröffentlichungstag und Bekanntmachung des Hinweises auf die Patenterteilung: **22.04.1998**
(21) Anmeldenummer: 94912531.4
(22) Anmeldetag: 24.03.1994
(51) Int. Cl.: C07J 43/00, C07J 41/00, A61K 31/58, A61K 31/57

(54) **NEUE GLUCOCORTICOIDE**
NEW GLUCOCORTICOIDS
GLUCOCORTICOIDES NOUVEAUX

(30) Priorität: 07.04.1993 DE 4311987
(43) Veröffentlichungstag der Anmeldung: 24.01.1996
(73) Patentinhaber: SCHERING AKTIENGESELLSCHAFT, 13342 Berlin (DE)
(72) Erfinder: ZENTEL, Hans Joachim, 13465 Berlin (DE); TÖPERT, Michael, 13465 Berlin (DE); LAURENT, Henry, 13467 Berlin (DE); BRUMBY, Thomas, 10827 Berlin (DE); ESPERLING, Peter, 12107 Berlin (DE)
(86) Internationale Anmeldenummer: EP9400937
(87) Internationale Veröffentlichungsnummer: WO9422898

(56) Entgegenhaltungen:
- EP-A- 0 126 685
- WO-A-89/11859
- CHEMICAL ABSTRACTS, vol. 115, no. 7, 19. August 1991, Columbus, Ohio, US; abstract no. 072168, RYAKHOVSKAYA M I ET AL 'Synthesis and pharmacological activity of 21-esters of prednisolone containing glycine and glutamic acid'
- CHEMICAL ABSTRACTS, vol. 113, no. 13, 24. September 1990, Columbus, Ohio, US; abstract no. 108904, RYAKHOVSKAYA M I ET AL 'Synthesis and study of pharmacological activity of 21-esters of hydrocortisone with glycine and glutamic acid'
- CHEMICAL ABSTRACTS, vol. 118, no. 17, 26. April 1993, Columbus, Ohio, US; abstract no. 169434, HORI K ET AL '21-Substituted steroids'
- ANALYTICAL BIOCHEMISTRY, Bd.99, Nr.1, 15. Oktober 1979, NEW YORK US Seiten 53 - 64 M. J. CASTILLO ET AL 'Sensitive Substrates for Human Leukocyte Elastase and Porcine Pancreatic Elastase: A Study of the Merits of Various Chromophoric and Fluorogenic Leaving Groups in Assays for Serine Proteases' in der Anmeldung erwähnt

## Beschreibung

Die Erfindung betrifft Glucocorticoide der allgemeinen Formel **I**

R-Val-O-GC (**I**),

worin
O-GC der Rest eines antiinflammatorisch wirksamen 21-Hydroxycorticoids ist,
Val einen in der 21-Position des Corticoids befindlichen Valinrest darstellt und
R ein Wasserstoffatom oder einen gegebenenfalls durch Hydroxygruppen, Aminogruppen, Oxogruppen und/oder Halogenatome substituierten und/oder durch Sauerstoffatome, SO₂-Gruppen und/oder NH-Gruppen unterbrochener Kohlenwasserstoffrest mit bis zu 32 Kohlenstoffatomen bedeutet
und deren Salze.

Die erfindungsgemäßen Glucocorticoide sind wertvolle Zwischenprodukte und/oder pharmakologisch wirksame Substanzen, die unter anderem zur Behandlung von entzündlichen Zuständen, die von einer erhöhten Aktivität des Enzyms Leukozyten-Elastase gekennzeichnet sind, dienen.

Glucocorticoide sind die am besten bekannte Klasse antiinflammatorischer Wirkstoffe. Aufgrund ihres breiten Einsatzbereiches und ihrer starken antiinflammatorischen Wirksamkeit sind Corticoid-Präparate Therapeutika der ersten Wahl in einer Vielzahl von entzündlichen Erkrankungen, wie etwa Erkrankungen des rheumatoiden Formenkreises, Allergien, entzündliche Lungen-, Herz- und Darmerkrankungen, Asthma bronchiale, hyperproliferative Erkrankungen der Haut (Psoriasis), Ekzeme, Autoimmunerkrankungen oder Schockzustände.
Ihre potentiellen Nebenwirkungen, wie Unterdrückung der Gehirn-Hypophysen-Nebennieren-Achse, ihre katabole Wirksamkeit, ihr Einfluß auf Salz- und Wasserhaushalt, Osteoporose, ihr Einfluß auf den Blutzuckerspiegel bei Diabetikern oder die Induktion von Hautatrophie bei lokaler Applikation, verhindern einen noch breiteren therapeutischen Einsatz dieser Substanzklasse. Nach dem heutigen Kenntnisstand werden diese Nebenwirkungen, ebenso wie die antientzündliche Wirkung der Glucocorticoide, durch den gleichen Rezeptor vermittelt. Bis dato sind daher Verringerungen des Nebenwirkungspotentials durch Erhöhung der metabolischen Clearance von lokal wirksamen Corticoiden erzielt worden (Antedrug-Prinzip). Lipophilere Prodrugs sollen die Penetration von Corticoiden in die Haut fördern und die Retention von Corticoiden in der Lunge verbessern. Trotz des verringerten Nebenwirkungspotentials moderner Corticoide bleiben insbesondere langfristige Therapien mit Wirkstoffen dieser Substanzklasse kritisch.
Die Leukozyten-Elastase ist eine Serin-Protease mit einem Molekulargewicht von etwa 30.000 D. Sie wird in Promyelozyten gebildet und findet sich in hauptsächlich in neutrophilen Granulozyten [Duswald, K. H. (1983). Zur Pathobiochemie der Leukozyten-Elastase, GIT Verlag Ernst Giebler, Darmstadt.]. Ihr Vorkommen ist auch in Monozyten, Lymphozyten und eosinophilen Granulozyten beschrieben [Kargi et al. (1990). Elastase and Cathepsin G of human monocytes: Heterogeneity and subcellular localication to peroxidase-positive granules. The Journal of Histochemistry and Cytochemistry 38: 1179-1186; Lungarella et al. (1992). Identification of elastase in human eosinophils: Immunolocalization, isolation, and partial characterization. Archives of Biochemistry and Biophysics, 292: 128-135; Bristow et al. (1991). Elastase is a constituent product of T cells. Biochemical and Biophysical Research Communications, 181: 232-239.] Das Enzym wird nach Stimulation von Granulozyten und Monozyten *in vitro* sezerniert [Schmidt (1978). Differential release of elastase and chymotrypsin from polymorphonuclear leukocytes. In: Neutral Proteases of Human Polymorphonuclear Leukocytes. Havemann and Janoff (Hrsg.) Urban & Schistzenberg Inc. Baltimore, München; Xie et al. (1993). Release of elastase from monocytes adherent to a fibronectin-gelatin surface. Blood 81: 186-192.]. *In vivo* werden hohe Elastase-Aktivitäten bei entzündlichen Erkrankungen der Lunge, bei Rhinitis, in Synovialflüssigkeit bei rheumatoider Arthrtis und auf der Hautoberfläche bei verschiedenen Ekzemformen beobachtet [Tanaka et al. (1990). A sensitive and specific assay for granulocyte elastase in inflammatory tissue fluid using L-pyroglutamyl-L-prolyl-L-valine-p-nitroanilide. Clinica Chimica Acta 187: 173-180; Wiedow et al. (1992). Lesional elastase activity in psoriasis, contact dermatitis, and atopic dermatitis. Journal for Investigative Dermatology 99: 306-309.]. Elastase-Inhibitoren werden als Therapeutika für die Indikation Lungenemphysem entwickelt.
Leukozyten-Elastase spaltet Ester- und Peptidbindungen C-terminal einer kurzen Erkennungssequenz. Die Erkennungssequenz ist gut charakterisiert. Der C-terminale Rest spielt für die katalytische Aktivität des Enzyms eine geringe Rolle [Castillo et al. (1979). Sensitive substrates for human leukocyte and porcine pancreatic elastase: A study of the merits of various chromophoric and fluorogenic leaving groups in assays for serine proteases. Analytical Biochemistry 99: 53-64.].
Bekannt ist der Versuch, eine antientzündliche Wirkung durch Hemmung der Leukozyten-Elastase zu erzielen.
Ebenfalls bekannt ist, daß Elastase Transcortin, das Transportprotein von physiologischen, nur schwach antiinflammatorisch wirksamen Corticoiden (Hydrocortison, Corticosteron), spaltet. Das Spaltprodukt dieses Proteins besitzt eine deutlich verminderte Affinität zum Glucocorticoid. Darauf baut die Hypothese auf, daß durch die Aktivität der Leukozyten-Elastase eine entzündungsspezifische Freisetzung von Corticoiden bewirkt werden könnte [Hammond et al. (1990). A role for corticosteroidbinding globulin in delivery of cortisol to activated neutrophils. Joumal of Clinical Endocrinology and Metabolism 71: 3439; Hammond et al. (1990). Interaction between corticosteroid binding globulin and activated leukocytes in vitro. Biochemical and Biophysical Research Communications 172: 172-177.]. Neu ist die Idee, die enzymatische Aktivität von Leukozyten-Elastase zur entzündungsspezifischen Aktivierung inaktiver Corticoid-Prodrugs auszunutzen. Solche Verbindungen könnten das Nebenwirkungspotential von Glucocorticoiden, z. B. in dermatologischen Indikationen, Erkrankungen des rheumatoiden Formenkreises oder beim Einsatz von Glucocorticoiden in entzündlichen Lungenerkrankungen, reduzieren.
Durch die katalytische Aktivität von Leukozyten-Elastase werden die erfindungsgemäßen Glucocorticoid-Prodrugs in das wirksame Glucocorticoid und das pharmakologisch inaktive Peptid gespalten.
Auf diese Weise wird das aktive Glucocorticoid selektiv im Entzündungsherd freigesetzt. Dieser Umstand führt zu einer Erhöhung der Konzentration des aktiven Glucocorticoids im Entzündungsherd. Die Konzentrationen des aktiven Glucocorticoids in nichtentzündeten Bereichen werden somit minimal gehalten. Dadurch wird eine Verringerung des lokalen und systemischen Nebenwirkungspotentials erreicht.

Als erfindungsgemäße Glucocorticoid-Prodrugs können beispielsweise solche verwendet werden, die Derivate von 21-Hydroxycorticoiden sind, welche in der EPA 0098 566 oder der "Roten Liste" [Herausg. Bundesverband der Pharmazeutischen Chemie e. V. Frankfurt a. Main] erwähnt sind.
Als Reste R der Prodrugs kommen beispielsweise Acylreste mit 1 bis 12 Kohlenstoffatomen, Benzylreste, oder solche in Betracht, die mit dem Valinrest einen gegebenenfalls mit den üblichen Schutzgruppen versehenen Oligopeptidrest mit 2 bis 6 Aminosäuren bilden.
Bevorzugte Oligopeptidreste sind solche von aliphatischen Aminosäuren und insbesondere solche, die als Aminosäuren Alanin, Prolin und Valin enthalten.
Geeignete Schutzgruppen sind beispielsweise jene, die in Band XV/1 der "Methoden der Organischen Chemie [Houben-Weyl, S. 20 ff] aufgeführt sind.

Besonders bevorzugte Glucocorticoide sind erfindungsgemäß solche der allgemeinen Formel **II**. 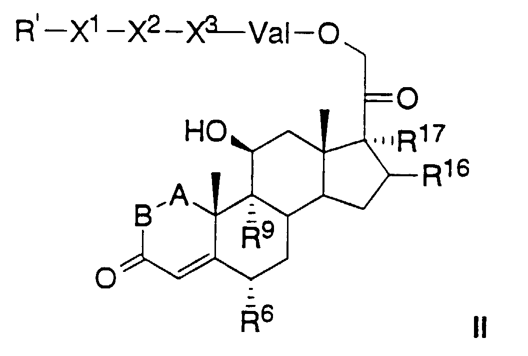 worin
- R'=: H, CH=O (C=O)R", (C=O)OR" oder SO₂R"
- X¹-X³ =: unabhängig voneinander Alanin, Prolin oder Valin,
- A-B =: CH₂-CH₂ oder CH=CH
- R⁶ =: H, F, Cl, Me,
- R⁹ =: H, F, Cl,
- R¹⁶ =: H, Me, OH,
- R¹⁷ =: H, OH, O(C=O)R"' oder
- R¹⁶ R¹⁷ =: Alkylidendioxy ist, in denen
R" ein C₁-C₁₈ enthaltendes Kohlenwasserstoff-Radikal und
R"' ein C₁-C₁₀ Alkyl (gerad- oder verzweigt-kettig) Aryl, Alkylaryl oder C₁-C₃ Alkoxy Radikal darstellen und
der Alkylidenrest sich von einem 1-6 C-Atome enthaltenden aliphatischen Aldehyd, einem 3-6 C-Atome enthaltendem Keton oder 5-6 C-Atome enthaltendem cyclischen Keton oder Benzaldehyd ableitet und deren Salze.
Die bevorzugte Erkennungssequenz besteht aus X¹-X³ = unabhängig voneinander Alanin, Prolin oder Valin und insbesondere aus X¹, X² = Alanin, X³ = Prolin, Valin. Verwendbar im Sinne der Erfindung ist allerdings auch jede andere Tetrapeptid-Sequenz, sofem sie an Elastase bindet und als Substrat akzeptiert wird.

Geeignete Kohlenwasserstoffradikale R" und R"' sind solche, die sich von den bereits erwähnten Schutzgruppen ableiten.
Die Synthesen der erfindungsgemäßen Glucocorticoide erfolgt nach den dem Fachmann wohlbekannten Verfahren, wie sie bei Peptidsynthesen allgemein angewendet werden ("Methoden der Organischen Chemie (Houben-Weyl, Bd XV/1 und XV/2). Die nachfolgenden Anwendungsbeispiele dienen zur näheren Erläuterung der Erfindung.

Die verwendeten Abkürzungen sind:
Aib = Aminoisobuttersäure, Ala = Alanin, Gly = Glycin, Pro = Prolin, Val = Valin;
BMV = Betamethason-17-valerat, FC = Fluocortolon, HC = Hydrocortison, MP = 6α-Methylprednisolon, MPP = 6α-Methylprednisolon-17-propionat, TCA = Triamcinolonacetonid;
Ac = Acetyl, ASA = Aminosäureanalyse, Boc = *tert.*-Butoxycarbonyl, Bz = Benzoyl, Cbs = 4Chlorbenzolsulfonyl, Cbp = 3-(9-Carbazolyl)-propionsaure, DDC = Dicyclohexylcarbodiimid, DMAP = 4-(N,N-Dimethylamino)pyridin, DPAc = Diphenylacetyl, DPC = Diphenylcarbamoyl, Fmoc = 9-Fluorenylmethoxycarbonyl, NCA = N-carbonic anhydride, Piv = Pivaloyl, Pht = Phthyloyl, TFA = Trifluoressigsäure, Z = Benzyloxycarbonyl.
Die IUPAC Empfehlungen für die Benennung von Peptiden [Biochem. J. **219**, 345 (1984)] werden in diesen Anwendungsbeispielen befolgt.

### Synthesen

### Beispiel 1

### N-(1,1-Dimethylethoxycarbonyl)-valin [11β,21-dihydroxy-3,20-dioxo-6α-methyl-17-propionyloxy-pregna-1,4-dien-21-yl]ester (Boc-Val-O-MPP)

Eine Lösung von 2.15 g (5.00 mmol) 6α-Methylprednisolon-17-propionat in 25 ml Dichlormethan wird mit 1.36 g (6.25 mmol) N-(1,1-Dimethylethoxycarbonyl)-valin und 121 mg (0.99 mmol) 4-Dimethylaminopyridin versetzt. Dann wird eine Lösung von 1.36 g (6.59 mmol) Dicyclohexylcarbodiimid in 5 ml Dichlormethan in einem Schuß zugegeben. Nach 1-3 Stunden wird der entstandene Niederschlag abgesaugt und mit Diethylether gewaschen. Das Filtrat wird im Vakuum eingeengt. Chromatographie an Kieselgel (Hexan→Hexan/Essigester 1:1) liefert 3.01 g (96 %) N-(1,1-Dimethylethoxycarbonyl)-valin [11β,21-dihydroxy-3,20-dioxo-6α-methyl-17-propionyloxy-pregna-1,4-dien-21-yl] ester. Kristallisation aus Dichlormethan/ Diisopropylether. Schmelzpunkt 110-130 °C. [α]_{D} = +32° (Chloroform).

### Beispiel 2

### Valin [11β,21-dihydroxy-3,20-dioxo-6α-methyl-17-propionyloxy-pregna-1,4- dien-21-yl] ester Trifluoracetat (H-Val-O-MPP-TFA)

896 mg (1.42 mmol) N-(1,1-Dimethylethoxycarbonyl)-valin [11β,21-dihydroxy-3,20-dioxo-6α-methyl-1 7-propionyloxy-pregna-1,4-dien-21-yl] ester werden mit 5 ml Trifluoressigsäure unter initialer Kühlung übergossen und 1-2 Stunden bei Raumtemperatur gerührt. Dann verdampft man die Trifluoressigsäure im Vakuum. Den Rückstand nimmt man in wenig Dichlormethan auf, gibt 5 ml Toluol hinzu und dampft anschließend im Vakuum zur Trockne ein. Der Rückstand wird kristallisiert. Man erhält 728 mg (80 %) Valin [11β,21-dihydroxy-3,20-dioxo-6α-methyl-17-propionyloxy-pregna-1,4dien-21-yl] ester-Trifluoracetat. Schmelzpunkt 148 °C.

### Beispiel 3

### N-(N-(N-(N-(1,1-Dimethylethoxycarbonyl)-L-alanyl)-L-alanyl)-L-prolyl)-L-valin [11β,2-dihydroxy-3,20-dioxo-6α-methyl-17-propionyloxy-pregna-1,4-dien-21-yl]ester (Boc-Ala-Ala-Pro-Val-O-MPP) (Boc-Ala-Ala-Pro-Val-O-MPP)

336 mg (0.52 mmol) Valin [11β,21-dihydroxy-3,21-dioxo-6α-methyl-17-propionyloxy-pregna-1,4-dien-21-yl]ester-Trifluoracetat, 180 mg (0.50 mmol) N-(1,1-Dimethylethoxycarbonyl)-alanyl-alanyl-prolin, 68 mg (0.50 mmol) Hydroxybenzotriazol werden in 20 ml Dichlormethan gelöst und 150 mg (0.72 mmol) Dicyclohexylcarbodiimid in 2 ml Dichlormethan zugegeben. Dann werden sofort 55 ml (0.50 mmol) N-Methylmorpholin zugesetzt und 2 Stunden bei Raumtemperatur gerührt. Zur Aufarbeitung wird vom Dicylohexylhamstoff abfiltriert, mit Diethylether nachgewaschen und die vereinigten organischen Phasen mit jeweils 40 ml 0.5 N HCI, 0.5 N NaOH und gesättigter Natriumchloridlösung gewaschen und über Natriumsulfat getrocknet. Verdampfen des Lösemittels im Vakuum ergibt das Rohprodukt. Chromatographie an Kieselgel (Hexan → Hexan/Aceton 1:1) ergibt 402 mg (89 %) N-(N-(N-(N-(1,1-Dimethylethoxycarbonyl)-L-alanyl)-L-alanyl)-L-prolyl)-L-valin [11β,21-dihydroxy-3,20-dioxo-6α-methyl-17-propionyloxy-pregna-1,4-dien-21-yl] ester. Kristallisation aus Dichlormethan/Diisopropylether/Hexan.
Schmp. Sintern ab 135 °C, Zersetzung ab 160 °C, [α]_{D} = -28° (c = 0.5% in Chloroform), HPLC: 99%.
ASA: Ala 2.04 Pro 0.95 Val 1.01, Racemtest (GC): D-Ala <1% D-Pro <1% D-Val <1%.

### Beispiel 4

### N-(N-(N-(N-(9H-Fluoren-9-ylmethoxycarbonyl)-L-alanyl)-L-alanyl)-L-prolyl)-L-valin [11β,21-dihydroxy-3,20-dioxo-6α-methyl-17-propionyloxy-pregna-1,4-dien-21-yl]ester (Fmoc-Ala-Ala-Pro-Val-O-MPP)

Wie in Beispiel 3 beschrieben werden 631 mg (0.98 mmol) Valin [11β,21-dihydroxy-3,21-dioxo-6α-methyl-17-propionyloxy-pregna-1,4-dien-21-yl] ester-Trifluoracetat, 446 mg (0.93 mmol) N-(9H-Fluoren-9-ylmethoxycarbonyl)-alanyl-alanyl-prolin, 136 mg (1.00 mmol) Hydroxybenzotriazol, 215 mg (1.04 mmol) Dicyclohexylcarbodiimid und 220 ml (2.00 mmol) N-Methylmorpholin umgesetzt. Chromatographie an Kieselgel (Dichlormethan → Dichlormethan/Methanol 97:3) ergibt 400 mg (42 %) N-(N-(N-(N-(9H-Fluoren-9-ylmethoxycarbonyl)-L-alanyl)-L-alanyl)-L-prolyl)-L-valin [11β,21-dihydroxy-3,20-dioxo-6α-methyl-17-propionyloxy-pregna-1,4-dien-21-yl]ester. Kristallisation aus Dichlormethan/Diisopropylether/ Hexan.
[α]_{D} = -24° (c = 0.5% in Chloroform), HPLC: 98%.
Racemtest (GC): D-Ala <1% D-Pro <1% D-Val <1%.

### Beispiel 5

### N-(N-(N-(N-(Phenylmethoxycarbonyl)-L-alanyl)-L-alanyl)-L-prolyl)-L-valin [11β,21-dihydroxy-3,20-dioxo-6α-methyl-17-propionyloxy-pregna-1,4-dien-21-yl]ester (Z-Ala-Ala-Pro-Val-O-MPP)

Unter den in Beispiel 3 angegebenen Bedingungen werden 131 mg (0.20 mmol) Valin [11β,21-dihydroxy-3,21-dioxo-6α-methyl-17-propionyloxy-pregna-1,4-dien-21-yl]ester-Trifluoracetat, 72 mg (0.19 mmol) N-(Benzyloxycarbonyl)-alanyl-alanyl-prolin (Z-Ala-Ala-Pro-OH), 51 mg (0.37 mmol) Hydroxybenzotriazol, 46 mg (0.22 mmol) Dicyclohexylcarbodiimid und 30 ml (0.28 mmol) N-Methyl-morpholin umgesetzL Gradientenchromatographie an Kieselgel (Dichlormethan → Dichlormethan/ Methanol 9:1) liefert 101 mg (60%) N-(N-(N-(N-(Phenylmethoxycarbony)-L-alanyl)-L-alanyl)-L-prolyl-L-valin [11β,21-dihydroxy-3,20-dioxo-6α-methyl-17-propionyloxy-pregna-1,4-dien-21-yl] ester. Kristallisation aus Methanol/Diisopropylether.
Schmp. ab 137° C (Zers.), [α]_{D} = -23° (c = 0.5% in Chloroform), HPLC: 98-99%.
Racemtest (GC): D-Ala 1.8% D-Pro <1% D-Val <1%.

### Beispiel 6

### N-(N-(N-(L-alanyl)-L-alanyl)-L-prolyl)-L-valin [11ß,21-dihydroxy-3,20-dioxo-6α-methyl-17-propionyloxy-pregna-1,4-dien-21-yl]ester Trifluoracetat (H-Ala-Ala-Pro-Val-O-MPP-TFA)

3.5 g (4.0 mmol) Boc-Ala-Ala-Pro-Val-O-MPP (Beispiel 3) werden in 50 ml Dichlormethan gelöst und mit 6.5 ml Trifluoressigsäure bei 0° C versetzt. Nach 2.5h wird das Lösemittel i. Vak. entfernt (5 HPA), der Rückstand in Dichlormethan aufgenommen und mit Diisopropylether gefällt. Man erhält 3.5 g (99%) N-(N-(N-(L-alanyl)-L-alanyl) -L-prolyl)-L-valin [11β,21-dihydroxy-3,20-dioxo-6α-methyl-17-propionyloxy-pregna-1,4-dien-21-yl] ester Trifluoracetat.
Schmp. 164 - 178° C (Zers.), [α]_{D} = -19° (c = 0.5% in Chloroform), HPLC: 98%.
ASA: Ala 1.93 Pro 1.09 Val 0.98, Racemtest (GC): D-Ala <1% D-Pro <1% D-Val <1%.

### Beispiel 7

### N-(N-(N-(N-(Methylcarbonyl)-L-alanyl)-L-alanyl)-L-prolyl)-L-valin[11β,21-dihydroxy-3,20-dioxo-6α-methyl-17-propionyloxy-pregna-1,4-dien-21-yl]ester (Ac-Ala-Ala-Pro-Val-O-M PP)

500 mg (0.57 mmol) H-Ala-Ala-Pro-Val-O-MPP x TFA (Beispiel 3) werden in 20 ml Dichlormethan gelöst, mit 115 µl (1.21 mmol) Acetanhydrid versetzt und 24h bei Raumtemperatur gerührt. Der nach Entfernung des Lösemittels i. Vak. verbleibende Rückstand wird chromatographiert (200g Kieselgel 60, Dichlormethan → Dichlormethan/Methanol 9:1). Man erhält 344 mg (75%) N-(N-(N-(N-(Methylcarbonyl)-L-alanyl)-L-alanyl)-L-prolyl)-L-valin [11β,21-dihydroxy-3,20-dioxo-6α-methyl-17-propionyloxy-pregna-1,4dien-21-yl] ester, und nach Umkristallisation 305 mg (66%) kristallin aus Methanol/Diisopropylether/Hexan.
Schmp. 152-155° C Sintern; 174° C, [α]_{D} = -28° (c = 0.5% in Chloroform), HPLC: 98-99%.
Racemtest (GC): D-Ala 2.3% D-Pro <1% D-Val <1%.

### Beispiel 8

### N-(N-(N-(N-(Phenylcarbonyl)-L-alanyl)-L-alanyl)-L-prolyl)-L-valin [11β,21-dihydroxy-3,20-dioxo-6α-methyl-17-propionyloxy-pregna-1,4-dien-21-yl]ester (Bz-Ala-Ala-Pro-Val-O-MPP)

250 mg (0.28 mmol) H-Ala-Ala-Pro-Val-O-MPP x TFA (Beispiel 3) werden in 50 ml Dichlormethan und 5 ml Triethylamin gelöst, mit 49 µl (0.37 mmol) Benzoylchlorid versetzt und 28h bei Raumtemperatur gerührt. Zur Aufarbeitung wird mit Wasser und gesättigter Natriumchlorid-Lösung gewaschen, über Natriumsulfat getrocknet und i.Vak. eingeengt. Der nach Entfemung des Lösemittels i. Vak. verbleibende Rückstand wird chromatographiert. Gradienten-Chromatographie an 200g Kieselgel 60 (Aceton/Hexan 1:2 → 2:1) ergibt 153 mg (62%) N-(N-(N-(N-(Phenylcarbony(-L-alanyl)-L-alanyl)-L-prolyl)-L-valin [11β,21-dihydroxy-3,20-dioxo-6α-methyl-17-propionyloxy-pregna-1,4-dien-21-yl] ester, und nach Umkristallisation 131 mg (53%) kristallin aus Dichlormethan/Diisopropylether.
Schmp. ab 150° C Zersetzung, [α]_{D} = -14° (c = 0.5% in Chloroform), HPLC: 95-98%.
Racemtest (GC): D-Ala 5.0% D-Pro <1% D-Val <1%.

### Beispiel 9

### N-(N-(N-(N-(Valeroyl)-L-alanyl)-L-alanyl)-L-prolyl)-L-valin [11β,21-dihydroxy-3,20-dioxo-6α-methyl-17-propionyloxy-pregna-1,4-dien-21-yl] ester (Valeroyl-Ala-Ala-Pro-Val-O-M PP)

307 mg (0.35 mmol) H-Ala-Ala-Pro-Val-O-MPP x TFA (Beispiel 3), 36 µl (0.33 mmol) Valeriansäure, 52 mg (0.35 mmol) N-Hydroxy-benzotriazol und 74 µl (0.66 mmol) N-Methyl-morpholin werden in 40 ml Dichlormethan gelöst, mit einer Lösung von 84 mg (0.41 mmol) Dicyclohexylcarbodiimid in 10 ml Dichlormethan versetzt und 4h bei Raumtemperatur gerührt. Zur Aufarbeitung wird der ausgefallene Harnstoff abfiltriert, das Filtrat mit 40 ml Dichlormethan verdünnt und mit je 40 ml 0.5 N NaOH, 0.5 N HCI und gesättigter Natriumchlorid-Lösung gewaschen, über Natriumsulfat getrocknet und i.Vak. eingeengt. Der nach Entfernung des Lösemittels i. Vak. verbleibende Rückstand wird chromatographiert. Gradienten-Chromatographie an 200g Kieselgel 60 (Dichlormethan → Dichlormethan/Methanol 95:5) ergibt 180 mg (64%) N-(N-(N-(N-(Valeroyl)-L-alanyl)-L-alanyl)-L-prolyl)-L-valin [11β,21-dihydroxy-3,20-dioxo-6α-methyl-17-propionyloxy-pregna-1,4-dien-21-yl] ester, und nach Umkristallisation 157 mg (56%) kristallin aus Dichlormethan/Diisopropylether/Hexan.
Schmp. 142° C, [α]_{D} = -54° (c = 0.5% in Methanol), HPLC: 9498%.
Racemtest (GC): D-Ala 1.7% D-Pro <1% D-Val <1%.

### Beispiel 10

### N-(N-(N-(N-((Diphenylmethyl)carbonyl)-L-alanyl)-L-alanyl)-L-prolyl)-L-valin [11β,21-dihydroxy-3,20-dioxo-6α-methyl-17-propionyloxy-pregna-1,4-dien-21- yl] ester (DPAc-Ala-Ala-Pro-Val-O-MPP)

150 mg (0.17 mmol) H-Ala-Ala-Pro-Val-O-MPP x TFA (Beispiel 3), 40 mg (0.19 mmol) Diphenylessigsäure, 23 mg (0.17 mmol) N-Hydroxy-benzotriazol und 41 µl (0.66 mmol) Diisopropylethylamin werden in 3 ml Dichlormethan gelöst, mit einer Lösung von 69 mg (0.33 mmol) Dicyclohexylcarbodiimid in 4 ml Dichlormethan versetzt und 23h bei Raumtemperatur gerührt Zur Aufarbeitung wird der ausgefallene Harnstoff abfiltriert und der nach Entfemung des Lösemittels i. Vak. verbleibende Rückstand chromatographiert (Gradienten-Chromatographie, 24g Kieselgel 60, Hexan → Hexan/Aceton 1:2). Man erhält 148 mg (91%) N-(N-(N-(N-((Diphenylmethyl)carbonyl)-L-alanyl)-L-alanyl)-L-prolyl)-L-valin [11β,21-dihydroxy-3,20-dioxo-6α-methyl-17-propionyloxy-pregna-1,4dien-21-yl] ester, der nochmals an einer Lichroprep Si60A Fertigsäule (Hexan → Hexan/Dichlormethan/Methanol 30:60:10) gereinigt wird (86 mg 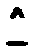 53%) und man erhält 75 mg (46%) kristalline Verbindung aus Dichlormethan/Diisopropylether.
Schmp. Sintert ab 157° C, 177° C, HPLC: 99%.
ASA: Ala 1.90 Pro 1.10 Val 1.00, Racemtest (GC): D-Ala 2.9% D-Pro 2.1% D-Val 0.8%.

### Beispiel 11

### N-(N-(N-(N-(Diphenylcarbamoyl)-L-alanyl)-L-alanyl)-L-prolyl)-L-valin [11β,21-dihydroxy-3,20-dioxo-6α-methyl-17-propionyloxy-pregna-1,4-dien-21-yl] ester (DPC-Ala-Ala-Pro-Val-O-MPP)

150 mg (0.17 mmol) H-Ala-Ala-Pro-Val-O-MPP x TFA (Beispiel 3) und 150 µl (0.89 mmol) Diisopropylethylamin werden in 6 ml Dichlormethan gelöst, mit 155 mg (0.37 mmol) Diphenylcarbamoylchlorid versetzt und 110h bei Raumtemperatur gerührt. Der nach Entfernung des Lösemittels i. Vak. verbleibende Rückstand wird chromatographiert. Gradienten-Chromatographie (Lichroprep Si 60 A Fertigsäule, Hexan → Hexan/Dichlormethan/Methanol 30:60:10) ergibt 158 mg (97%) N-(N-(N-(N-(Diphenylcarbamoyl)-L-alanyl)-L-alanyl)-L-prolyl)-L-valin[11β,21-dihydroxy-3,20-dioxo-6α-methyl-17-propionyloxy-pregna-1,4-dien-21-yl] ester, und nach Umkristallisation 112 mg (68%) kristallin aus Dichlormethan/Diisopropylether.
Schmp. 150 - 152° C, HPLC: 95.6 - 98.2%, Racemtest (GC): D-Ala 2.9% D-Pro 2.0% D-Val <1%.

### Beispiel 12

### N-(N-(N-(N-(2-Naphthoyl)-L-alanyl)-L-alanyl)-L-prolyl)-L-valin [11β,21-dihydroxy-3,20-dioxo-6α-methyl-17-propionyloxy-pregna-1,4-dien-21-yl] ester (2-Naphthoyl-Ala-Ala-Pro-Val-O-M PP)

120 mg (0.14 mmol) H-Ala-Ala-Pro-Val-O-MPP x TFA (Beispiel 3), 28 mg (0.16 mmol) 2-Naphthoesäure, 19 mg (0.14 mmol) N-Hydroxy-benzotriazol und 100 µl (0.57 mmol) Diisopropylethylamin werden in 3 ml Dichlormethan gelöst, mit einer Lösung von 67 mg (0.33 mmol) Dicyclohexylcarbodiimid in 1 ml Dichlormethan versetzt und 18h bei Raumtemperatur gerührt. Zur Aufarbeitung wird der ausgefallene Harnstoff abfiltriert und der nach Entfernung des Lösemittels i. Vak. verbleibende Rückstand chromatographiert (Gradienten-Chromatographie an 23g Kieselgel 60, Aceton/Hexan 1:1 → 2:3). Man erhält 103 mg (82%) N-(N-(N-(N-(2-Naphthoyl)-L-alanyl)-L-alanyl)-L-prolyl)-L-valin [11β,21--dihydroxy-3,20-dioxo-6α-methyl-17-propionyloxy-pregna-1,4-dien-21-yl] ester, und nach Umkristallisation 87 mg (69%) kristallin aus Dichlormethan/Diisopropylether.
Schmp. 166-170°C, HPLC: 97.6 - 97.9%, ASA: Ala 1.90 Pro 1.12 Val 0.98, Racemtest (GC): D-Ala 7.3% D-Pro 2.0% D-Val 0.8%.

### Beispiel 13

### N-(N-(N-(N-(3-Phenyl-butenoyl)-L-alanyl)-L-alanyl)-L-prolyl)-L-valin [11β,21-dihydroxy-3,20-dioxo-6α-methyl-17-propionyloxy-pregna-1,4-dien-21-yl] ester (Cinnamoyl-Ala-Ala-Pro-Val-O-M PP)

120 mg (0.14 mmol) H-Ala-Ala-Pro-Val-O-MPP x TFA (Beispiel 3), 24 mg (0.16 mmol) Zimtsäure, 19 mg (0.14 mmol) N-Hydroxy-benzotriazol und 100 µl (0.57 mmol) Diisopropylethylamin werden in 3.5 ml Dichlormethan gelöst, mit einer Lösung von 67 mg (0.33 mmol) Dicyclohexylcarbodiimid in 0.5 ml Dichlormethan versetzt und 64h bei Raumtemperatur gerührt. Zur Aufarbeitung wird der ausgefallene Harnstoff abfiltriert und der nach Entfemung des Lösemittels i. Vak. verbleibende Rückstand chromatographiert (23g Kieselgel 60, Dichlormethan/Aceton/Hexan 1:1:1 → Aceton/Hexan 2:1). Man erhält 98 mg (80%) N-(N-(N-(N-(3-Phenyl-butenoyl)-L-alanyl)-L-alanyl)-L-prolyl)-L-valin [11β,21-dihydroxy-3,20-dioxo-6a-methyl-17-propionyloxy-pregna-1,4-dien-21-yl] ester, und nach
Umkristallisation 81 mg (66%) kristalline Verbindung aus Essigester/Hexan.
Schmp. Sintern ab 154°, Schmelze bei 191°C, HPLC: 97.2 - 97.9%, ASA: Ala 1.89 Pro 1.13 Val 0.98, Racemtest (GC): D-Ala 10.2% D-Pro 3.6% D-Val 2.5%.

### Beispiel 14

### N-(N-(N-(N-(4-Chlorbenzolsulfonyl)-L-alanyl)-L-alanyl)-L-prolyl)-L-valin [11β,21-dihydroxy-3,20-dioxo-6α-methyl-17-propionyloxy-pregna-1,4-dien-21-yl] ester (Cbs-Ala-Ala-Pro-Val-O-MPP)

100 mg (0.11 mmol) H-Ala-Ala-Pro-Val-O-MPP x TFA (Beispiel 3) und 300 µl (1.71 mmol) Diisopropylethylamin werden in 5 ml Tetrahydrofuran gelöst, mit 238 mg (1.13 mmol) 4-Chlorbenzolsulfonsäure-chlorid versetzt und 23h bei Raumtemperatur gerührt Der nach Entfemung des Lösemittels i. Vak. verbleibende Rückstand wird chromatographiert Gradienten-Chromatographie an 25 g Kieselgel 60 (Hexan/Aceton 2:1 → 1:2) ergibt 75 mg (70%) N-(N-(N-(N-(4-Chlorbenzolsulfonyl)-L-alanyl)-L-alanyl)-L-prolyl)-L-valin [11β,21-dihydroxy-3,2-dioxo-6α-methyl-17-propionyloxy-pregna-1,4-dien-21-yl] ester, und nach Umkristallisation aus Dichlormethan/Diisopropylether 47 mg (44%) kristallines Produkt.
Schmp. Sintem ab 151°, Schmelze bei 195°C, HPLC: 97.2 98.0%, ASA: Ala 1.60 Pro 1.28 Val 1.12, Racemtest (GC): D-Ala 2.8% D-Pro 1.2% D-Val 0.8%.

### Beispiel 15

### N-(N-(N-(N-(2,2-Dimethylpropionyl)-L-alanyl)-L alanyl)-L-prolyl)-L-valin [11β,21-dihydroxy-3,20-dioxo-6α-methyl-17-propionyloxy-pregna-1,4-dien-21-yl] ester (Piv-Ala-Ala-Pro-Val-O-M PP)

150 mg (0.17 mmol) H-Ala-Ala-Pro-Val-O-MPP x TFA (Beispiel 3) und 60 µl (0.34 mmol) Diisopropylethylamin werden in 6 ml Dichlormethan gelöst, mit 20 µl (0.18 mmol) Pivaloylchlorid versetzt und 25h bei Raumtemperatur gerührt Der nach Entfernung des Lösemittels i. Vak. verbleibende Rückstand wird direkt chromatographiert. Gradienten-Chromatographie (24g Kieselgel 60, Hexan → Hexan/-Aceton 7:3) ergibt 102 mg (70%) leicht verunreinigten N-(N-(N-(N-(2,2-Dimethylpropionyl)-L-alanyl)-L-alanyl)-L-prolyl-L-valin [11β,21-dihydroxy-3,20-dioxo-6α-methyl-17-propionyloxy-pregna-1,4-dien-21-yl] ester. Eine zweite Chromatographie (Lichroprep Si 60 A Fertigsäule, Hexan/Dichlormethan 1:1 → Hexan/Dichlormethan/Methanol 30:60:10) ergibt 92 mg, 83 mg kristalline Verbindung aus Dichlormethan/ Diisopropylether.
Sintert ab 157° C, Schmp. 177°C, HPLC: 98.7%, ASA: Ala 1.90 Pro 1.10 Val 1.00, Racemtest (GC): D-Ala 2.6% D-Pro 1.8% D-Val 0.6%.

### Beispiel 16

### N-(N-(N-(N-(3-(Carbazol-9-yl)-propionyl)-L-alanyl)-L-alanyl)-L-prolyl)-L-valin [11β,21-dihydroxy-3,20-dioxo-6α-methyl-17-propionyloxy-pregna-1,4-dien-21-yl] ester (Cbp-Ala-Ala-Pro-Val-O-MPP)

150 mg (0.17 mmol) H-Ala-Ala-Pro-Val-O-MPP x TFA (Beispiel 3), 61 mg (0.26 mmol) Carbazolpropionsäure, 35 mg (0.26 mmol) N-Hydroxy-benzotriazol und 122 µl (070 mmol) Diisopropylethylamin werden in 5 ml Dichlormethan gelöst, mit 65 mg (0.34 mmol) Ethyl-3(dimethylamino)-propylcarbodiimid (EDC) versetzt und 67h bei Raumtemperatur gerührt. Zur Aufarbeitung das Lösemittel i. Vak. entfernt und der verbleibende Rückstand chromatographiert (23g Kieselgel 60, Aceton/Hexan 1:9 → 1:1). Man erhält 140 mg (83%) N-(N-(N-(N-(3-(Carbazol-9-yl)-propionyl)-L-alanyl)-L-alanyl)-L-prolyl)-L-valin [11β,21-dihydroxy-3,20-dioxo-6a-methyl-17-propionyloxy-pregna-1,4-dien-21-yl] ester, und nach Umkristallisation 99 mg (59%) kristallin aus Aceton/Hexan.
Schmp. Sintem ab 158°, Schmelze bei 182°C, HPLC: 95.8 - 96.3%, Racemtest (GC): D-Ala 2.0% D-Pro 2.1% D-Val 1.4%.

### Beispiel 17

### N-(N-(N-(N-(N-(Phthaloyl)-glycyl)-L-alanyl)-L-alanyl)-L-prolyl)-L-valin [11β,21-dihydroxy-3,20-dioxo-6α-methyl-17-propionyloxy-pregna-1,4-dien-21-yl] ester (Pth-Gly-Ala-Ala-Pro-Val-O-MPP)

120 mg (0.14 mmol) H-Ala-Ala-Pro-Val-O-MPP x TFA (Beispiel 3), 31 mg (0.15 mmol) Phthaloylglycin, 20 mg (0.15 mmol) N-Hydroxy-benzotriazol und 48 µl (0.27 mmol) Diisopropylethylamin werden in 2 ml Dichlormethan gelöst, mit einer Lösung von 56 mg (0.27 mmol) Dicyclohexylcarbodiimid in 1 ml Dichlormethan versetzt und 17h bei Raumtemperatur gerührt. Zur Aufarbeitung wird der ausgefallene Hamstoff abfiltriert und der nach Entfernung des Lösemittels i. Vak. verbleibende Rückstand chromatographiert (Gradienten-Chromatographie, 25g Kieselgel 60, Hexan/Aceton 1:1 → 1:2). Man erhält 68 mg (52%) N-(N-(N-(N-(N-(Phthaloyl)-glycyl)-L-alanyl)-L-alanyl)-L-prolyl)-L-valin [11β,21-dihydroxy-3,20-dioxo-6α-methyl-17-propionyloxy-pregna-1,4-dien-21-yl] ester, und hieraus 66 mg (51%) kristallines Produkt aus Dichlormethan/Diisopropylether.
Schmp. Zers. unter Gasentwicklung ab 165° C, HPLC: 97.2 - 98.4, ASA: Ala 1.89 Pro 1.12 Val 0.99, Racemtest (GC): D-Ala 1.1% D-Pro 0.8% D-Val 0.8% Gly identifiziert.

### Beispiel 18

### N-(N-(N-(N-(N-((9H-Fluoren-9-ylmethoxy)carbonyl)-2-amino-2-methyl-propionyl)-L-alanyl)-L-alanyl)-L-prolyl)-L-valin [11β,21-dihydroxy-3,20-dioxo-6α-methyl-17-propionyloxy-pregna-1,4-dien-21-yl] ester (Fmoc-Aib-Ala-Ala-Pro-Val-O-M PP)

200 mg (0.14 mmol) H-Ala-Ala-Pro-Val-O-MPP x TFA (Beispiel 3), 120 mg (0.34 mmol) Fmoc-Aib-NCA und 200 µl (1.14 mmol) Diisopropylethylamin werden in 10 ml Dichlormethan gelöst und 23h bei Raumtemperatur gerührt. Zur Aufarbeitung wird das Lösemittel i. Vak. entfernt und der verbleibende Rückstand chromatographiert (Gradienten-Chromatographie, 25g Kieselgel 60, Hexan → Hexan/ Aceton 1:2). Man erhält 135 mg (55%) N-(N-(N-(N-(N-((9H-Fluoren-9-ylmethoxy)carbonyl)-2-amino-2-methyl-propionyl)-L-alanyl)-L-alanyl)-L-prolyl)-L-valin [11β,21-dihydroxy-3,20-dioxo-6α-methyl-17-propionyloxy-pregna-1,4-dien-21-yl] ester, und nach Umkristallisation 102 mg (44%) nach zweimaliger Kristallisation aus Dichlormethan/Diisopropylether.
[α]_{D} = -23° (c = 0.26% in Chloroform), HPLC: 97 - 98%. Racemtest (GC): D-Ala 1.4% D-Pro 1.4% D.Val 1.7% Aib identifiziert.

### Beispiel 19

### N-(1,1-Dimethylethoxycarbonyl)-valin [11β,17,20-trihydroxy-3,20-dioxo-6α-methyl-pregna-1,4-dien-21-yl] ester (Boc-Val-O-MP)

Analog Beispiel 1 werden 1.94 g 6α-Methylprednisolon, 1.37 g N-(1,1-Dimethylethoxycarbonyl)-valin, 74 mg 4-Dimethylaminopyridin und 1.34 g Dicyclohexylcarbodiimid in 30 ml Dichlormethan/Dioxan 1:1 umgesetzt. Chromatographie an Kieselgel (Dichlormethan → Dichlormethan/Aceton 3:1) ergibt 2.58 g (87 %) N-(1,1-Dimethylethoxycarbonyl)-valin [11β,17,20-trihydroxy-3,20-dioxo-6α-methyl-pregna-1,4 dien-21-yl] ester. Kristallisation aus Dichlormethan/ Diisopropylether.
Schmelzpunkt 144-148 °C. [a]_{D} = +70° (Chloroform).

### Beispiel 20

### Valin [11β,17,20-trihydroxy-3,20-dioxo-6α-methyl-pregna-1,4-dien-21-yl] ester Trifluoracetat (H-Val-O-MP TFA)

806 mg N-(1,1-Dimethylethoxycarbonyl)-valin [11β,17,20-trihydroxy-3,20-dioxo-6α-methyl-pregna-1,4-dien-21-yl] ester (Beispiel 19) werden unter den im Beispiel 2 beschriebenen Bedingungen umgesetzt. Man erhält 616 mg (75 %) Valin [11β,17,20-trihydroxy-3,20-dioxo-6α-methyl-pregna-1,4-dien-21-yl] ester Trifluoracetat.
Schmelzpunkt 167-171 °C (Zersetzung). [α]_{D} = +95° (Methanol).

### Beispiel 21

### N-(N-(N-(N-(1,1-Dimethylethoxycarbonyl)-L-alanyl)-L-alanyl)-L-prolyl)-L-valin [11β,17,21-trihydroxy-3,20-dioxo-6α-methyl-pregna-1,4-dien-21-yl] ester (Boc-Ala-Ala-Pro-Val-O-MP)

Unter den in Beispiel 3 angegebenen Bedingungen werden 1.44 g (2.46 mmol) Valin [11β,17,20-trihydroxy-3,20-dioxo-6α-methyl-pregna-1,4-dien-21-yl] ester Trifluoracetat, 800 mg (2.24 mmol) N-(1,1-Dimethylethoxycarbonyl)-alanyl-alanyl-prolin, 300 mg (2.24 mmol) Hydroxybenzotriazol, 555 mg (2.69 mmol) Dicyclohexylcarbodiimid und 0.49 ml (4.48 mmol) N-Methylmorpholin umgesetzt. Chromatographie an Kieselgel (Dichlormethan → Dichlormethan/Methanol 95:5) liefert 1.54 g (77%) N-(N-(N-(N-(1,1-Dimethylethoxycarbonyl)-L-alanyl)-L-alanyl)-L-prolyl)-L-valin [11β,17,21 -trihydroxy3,20-dioxo-6α-methyl-pregna-1,4-dien-21-yl] ester. Kristallisation aus Dichlormethan/Diisopropylether. Schmp. 155 - 175 °C, Zersetzung unter Gasentwicklung, [α]_{D} = -2° (c = 0.5% in Chloroform), HPLC: 96.5 - 97.5%. Racemtest (GC): D-Ala 3.2% D-Pro 2.3% D-Val 1.9%.

| | | | |
|---|---|---|---|
| Ber. | C 63.53 | H 7.93 | N 6.89 |
| Gef. | C 63.52 | H 7.70 | N 6.97 |

### Beispiel 22

### N-(N-(N-(N-((9H-Fluoren-9-yl-methoxycarbonyl)-L-alanyl)-L-alanyl)-L-prolyl)-L-valin [11β,17,21-trihydroxy-3,20-dioxo-6α-methyl-pregna-1,4-dien-21-yl] ester (Fmoc-Ala-Ala-Pro-Val-O-MP)

510 mg (0.87 mmol) Valin [11β,17,20-trihydroxy-3,20-dioxo-6α-methyl-pregna-1,4-dien-21-yl] ester Trifluoracetat, 347 mg (0.72 mmol) Fmoc-Ala-Ala-Pro-OH und 97 mg (0.72 mmol) N-Hydroxybenzotriazol werden in dieser Reihenfolge in 25 mL Dichlormethan gelöst mit 160 µL(1.45 mmol) N-Methyl-morpholin zugegeben. Dann wird eine Lösung von 298 mg (1.45 mmol) Dicyclohexylcarbodiimid in 5 mL Dichlormethan versetzt und 5h bei Raumtemperatur gerührt. Nach Kühlung auf -20° C wird vom ausgefallenen Harnstoff abgesaugt, mit Diethylether gewaschen die vereinigten Filtrate mit je 2 x 50 ml 0.5 N HCI, 0.5 N NaOH extrahiert, mit gesättigter Natriumchlorid-Lösung gewaschen und über Natriumsulfat getrocknet. Der nach Entfernung des Lösemittels i. Vak. verbleibende Rückstand wird direkt chromatographiert (110 g Kieselgel, Dichlormethan → Dichlormethan/Methanol 9:1). Man erhält 478 mg (71%) N-(N-(N-(N-((9H-Fluoren-9-yl-methoxycarbonyl)-L-alanyl)-L-alanyl)-L-prolyl)-L-valin [11β,17,21-trihydroxy-3,20-dioxo-6α-methyl-pregna-1,4-dien-21-yl] ester. Kristallisation aus Dichlormethan/Diisopropylether ergibt 449 mg.
Schmp. 169 -171 °C, [α]_{D} = -2° (c = 0.5% in Chloroform), HPLC: 96.1 - 96.8%. Racemtest (GC): D-Ala 2.5% D-Pro >1% D-Val 1.4%.

| | | | | |
|---|---|---|---|---|
| Ber. | C 68.07 | H 7.11 | N 5.99 | O 18.82 |
| Gef. | C 67.95 | H 7.54 | N 5.68 | O 18.74 |

### Beispiel 23

### N-(N-(N-(L-Alanyl)-L-alanyl)-L-prolyl)-L-val1n [11β,17,21-trihydroxy-3,20-dioxo-6α-methyl-pregna-1,4-dien-21-yl] ester Hydrochlorid (H-Ala-Ala-Pro-Val-O-MP x HCI)

939 mg (1.15 mmol) Boc-Ala-Ala-Pro-Val-O-MP (Beispiel 21) werden in 10 ml Dioxan gelöst und 10 ml HCI (4N in Dioxan) zugegeben. Nach 18h bei Raumtemperatur wird das Lösemittel i. Vak. entfernt, der Rückstand mit Dichlormethan verrieben, abgesaugt und die erhaltenen Kristalle aus Methanol/Diisopropylether umkristallisiert. Man erhält 835 mg (94%) N-(N-(N-(L-Alanyl)-L-alanyl)-L-prolyl)-L-valin [11β,17,21-trihydroxy-3,20-dioxo-6α-methyl-pregna-1,4-dien-21-yl] ester Hydrochlorid.
Schmp. ab 200° C (Zers.), [α]_{D} = -10° (c = 0.5% in Methanol), HPLC: 97%, Racemtest (GC): D-Ala 2.7% D-Pro 1.7% D-Val>1%.

### Beispiel 24

### N-(N-(N-(N-(Phenylcarbonyl)-L-alanyl)-L-alanyl)-L-prolyl)-L-valin [11β,17,21-trihydroxy-3,20-dioxo-6α-methyl-pregna-1,4-dien-21-yl] ester (Bz-Ala-Ala-Pro-Val-O-MP)

370 mg (0.46 mmol) Boc-Ala-Ala-Pro-Val-O-MP (Beispiel 21) werden in 3 ml Dioxan gelöst und 4 ml HCI (4N in Dioxan) zugegeben. Nach 16h bei Raumtemperatur wird das Lösemittel i. Vak. entfernt, in 4 ml Dichlormethan gelöst, mit 175 µl (1.00 mmol) Diisopropylethylamin und 58 µl (0.50 mmol) Benzoylchlorid versetzt und 17h bei Raumtemperatur gerührt. Zur Aufarbeitung wird das Lösemittel i. Vak. entfernt und der verbleibende Rückstand aus Dichlormethan/Diisopropylether kristallisiert. Man erhält 139 mg (37%) N-(N-(N-(N-(Phenylcarbonyl)-L-alanyl)-L-alanyl)-L-prolyl)-L-valin [11β,17,21-trihydroxy-3,20-dioxo-6α-methyl-pregna-1,4-dien-21-yl] ester.
HPLC: 97.1 - 97.7%, Racemtest (GC) : D-Ala 3.8% D-Pro 2.7% D-Val 2.6%.

### Beispiel 25

### N-(N-(N-(N-((Phenylmethoxy)carbonyl)-L-alanyl)-L-alanyl)-L-prolyl)-L-valin [11β,17,21-trihydroxy-3,20-dioxo-6α-methyl-pregna-1,4-dien-21-yl] ester (Z-Ala-Ala-Pro-Val-O-MP)

200 mg (0.27 mmol) H-Ala-Ala-Pro-Val-O-MP x HCI (Beispiel 23) und 37 µl (0.27 mmol) Triethylamin werden in 10 ml Dichlormethan gelöst, mit 42 µl (0.30 mmol) Chlorameisensäurebenzylester versetzt und 36h bei Raumtemperatur gerührt. Zur Aufarbeitung wird mit 20 ml Dichlormethan verdünnt und mit 2N Kaliumhydrogensulfat-Lösung und gesättigter Natriumchlorid-Lösung gewaschen, über Natriumsulfat getrocknet und l.Vak. eingeengt. Der nach Entfernung des Lösemittels i. Vak. verbleibende Rückstand wird chromatographiert. Gradienten-Chromatographie an 110g Kieselgel 60 (Dichlormethan → Dichlormethan/Methanol 9:1) ergeben 156 mg (69%) N-(N-(N-(N-((Phenylmethoxy)-carbonyl)-L-alanyl)-L-alanyl)-L-prolyl)-L-valin [11β,17,21-trihydroxy-3,20-dioxo-6α-methyl-pregna-1,4-dien-21-yl] ester, und nach Umkristallisation 123 mg (54%) kristallin aus Methanol/Diisopropylether.
HPLC: 98.7 - 99.7%, ASA: Ala 2.00 Pro 1.01 Val 0.99, Racemtest (GC): D-Ala 2.2% D-Pro <1% D-Val 1.1%.

### Beispiel 26

### N-(N-(N-(N-(Valeroyl)-L-alanyl)-L-alanyl)-L-prolyl)-L-valin[11β,17,21-trihydroxy-3,20-dioxo-6α-methyl-pregna-1,4-dien-21-yl] ester (Valeroyl-Ala-Ala-Pro-Val-O-MP)

200 mg (027 mmol) H-Ala-Ala-Pro-Val-O-MP x HCI **41c**, 33 µl (0.30 mmol) Valeriansäure, 41 mg (0.30 mmol) N-Hydroxy-benzotriazol und 66 µl (0.60 mmol) N-Methyl-morpholin werden in 20 ml Dichlormethan gelöst, mit einer Lösung von 123 mg (0.60 mmol) Dicyclohexylcarbodiimid in 5 ml Dichlormethan versetzt und 105h bei Raumtemperatur gerührt. Zur Aufarbeitung wird der ausgefallene Harnstoff abfiltriert und das Filtrat i.Vak. eingeengt. Gradienten-Chromatographie der Rohmenge an 110g Kieselgel 60 (Hexan → Hexan/Aceton 6:4) ergibt 139 mg (65%) N-(N-(N-(N-(Valeroyl)-L-alanyl)-L-alanyl)-L-prolyl)-L-valin [11β,17,21-trihydroxy-3,20-dioxo-6α-methyl-pregna-1,4-dien-21-yl] ester, 74 mg (35%) kristallin aus Methanol/Diisopropylether.
HPLC: 96.3-97.0%, Racemtest (GC): D-Ala 2.5% D-Pro 2.4% D-Val <1%.
ZK 162494 AZ 204436 ON 128

### Beispiel 27

### (2S)-2-((1,1-Dimethylethoxycarbonyl)-amino)-3-methyl-buttersäure [11β,17,21-trihydroxy-3,20-dioxo-pregn-4-en-21-yl] ester (Boc-Val-O-HC)

Eine Lösung von 3.63 g (10 mmol) Hydrocortison in 150 ml Dichlormethan wird mit 2.34 g (10.8 mmol) N-(tert.-Butoxycarbonyl)-valin, 500 mg (4.1 mmol) 4-Dimethylaminopyridin und 3.1 g (15 mmol) Dicyclohexylcarbodiimid versetzt. Die Lösung wird 3 Stunden bei Raumtemperatur gerührt, der entstandene Niederschlag abgesaugt und mit Dichlormethan gewaschen. Das Filtrat wird im Vakuum eingeengt. Die Chromatographie an Kieselgel (Hexan - Hexan/Essigester 1:1) liefert 2.87 g (51 %) (2S)-2-((1,1-Dimethylethoxycarbonyl)-amino)-3-methyl-buttersäure [11β,17,21-trihydroxy-3,20-dioxo-pregn-4-en-21-yl] ester. Kristallisation aus Dichlormethan/Diisopropylether.
Schmelzpunkt 169 °C. [α]_{D} = +97° (Chloroform).

### Beispiel 28

### (2S)-2-Amino-3-methyl-buttersäure [11β,17,21-trihydroxy-3,20-dioxo-pregn-4-en-21-yl] ester Trifluoracetat (H-Val-O-HC-TFA)

500 mg (0.89 mmol) (2S)-2-(((1,1-Dimethylethoxycarbonyl)-amino)-3-methyl-buttersäure [11β,17,21-trihydroxy-3,20-dioxo-pregn-4-en-21-yl] ester (Beispiel 27) werden mit 1 ml Trifluoressigsäure versetzt und 10 Minuten bei Raumtemperatur gerührt. Danach verdampft man die Trifluoressigsäure im Vakuum. Der Rückstand wird unter Rühren mit wenig Diethylether versetzt, der entstandene weiße Niederschlag abgesaugt und getrocknet. Man erhält 385 mg (76 %) (2S)-2-Amino-3-methyl-buttersäure [11β,17,21-trihydroxy-3,20-dioxo-pregn-4-en-21-yl] ester Trifluoracetat.
Schmelzpunkt 188 °C.

### Beispiel 29

### N-(N-(N-(N-(9H-Fluoren-9-yl-methoxycarbonyl)-L-alanyl)-L-alanyl)-L-prolyl)-L-valin [11β,17,21-trihydroxy-3,20-dioxo-pregn-4-en-21-yl] ester (Fmoc-Ala-Ala-Pro-Val-O-HC)

Eine Lösung von 350 mg (0.62 mmol) (2S)-2-Amino-3-methyl-buttersäure [11β,17,21-trihydroxy-3,20-dioxo-pregn-4-en-21-yl] ester Trifluoracetat (Beispiel 28) in 30 ml Dichlormethan wird mit 350 mg (0.73 mmol) N-(9-Fluorenylmethoxycarbonyl)-alanyl-alanyl-prolin, 100 mg (0.74 mmol) Hydroxybenzotriazol, 150 mg (0.72 mmol) Dicyclohexylcarbodiimid und 0.07 ml (0.63 mmol) N-Methylmorpholin versetzt und 2 Stunden bei Raumtemperatur gerührt. Zur Aufarbeitung wird vom Dicylohexylhamstoff abfiltriert, mit Diethylether nachgewaschen. Die vereinigten organischen Phasen werden mit jeweils 50 ml 0.5 N HCI, 0.5 N NaOH und gesättigter Natriumchloridlösung gewaschen und über Natriumsulfat getrocknet. Verdampfen des Lösemittels im Vakuum ergibt das Rohprodukt. Die Chromatographie an Kieselgel (Hexan → Hexan/Aceton 1:1) ergibt 405 mg (72 %) N-(N-(N-(N-(9H-Fluoren-9-yl-methoxycarbonyl)-L-alany) -L-alanyl)-L-prolyl)-L-valin [11β,17,21-trihydroxy-3,20-dioxo-pregn-4-en-21-yl] ester. Kristallisation aus Hexan/Essigester.
Schmelzpunkt 193 °C.

### Beispiel 30

### (2S)-2-((1,1-Dimethylethoxycarbonyl)-amino)-3-methyl-buttersäure [11β,21-dihydroxy-6α-fluor-16α-methyl-3,20-dioxo-pregna-1,4-dien-21-yl] ester (Boc-Val-O-FC)

26 mmol Fluocortolon (FC) werden mit 32 mmol Boc-Valin (Boc-Val-OH), 2.9 mmol 4-Dimethylaminopyridin und 34 mmol Dicyclohexylcarbodiimid in Dichlormethan/Dioxan 3:2 umgesetzt. Chromatographie (Essigester/Hexan 2:1): F₁ 9.3 g reines, F₂ 4.8 g leicht verunreinigtes Produkt. (Gesamtausbeute 92%) Kristallisation von F₁ aus Essigester/Hexan ergibt 7.0 g (46%) (2S)-2-((1,1-Dimethylethoxycarbonyl)-amino)-3-methyl-buttersäure [11β,21-dihydroxy-6α-fluor-16α-methyl-3,20-dioxo-pregna-1,4-dien-21-yl] ester.
Schmp. 166 - 168° C, [α]_{D} = +85° (c = 0.5% in Chloroform).

| | | | | |
|---|---|---|---|---|
| Ber. | C 66.88 | H 7.89 | N 2.44 | F 3.31 |
| Gef. | C 66.90 | H 7.82 | N 2.73 | F 3.29 |

### Beispiel 31

### (2S)-2-Amino-3-methyl-buttersäure [11β,21-dihydroxy-6α-fluor-16α-methyl-3,20-dioxo-pregna-1,4-dien-21-yl] ester Trifluoracetat (H-Val-O-FC x TFA)

6.4 g (11 mmol) Boc-Val-O-FC (Beispiel 29) werden mit Trifluoressigsäure/Dichlormethan analog Beispiel 2 umgesetzt. Kristallisation aus Dichlormethan/Diisopropylether (Ultraschallbad) ergibt 5.6 g (86%) (2S)-2-Amino-3-methyl-buttersäure [11β,21-dihydroxy-6α-fluor-16α-methyl-3,20-dioxo-pregna-1,4-dien-21-yl] ester Trifluoracetat.
Schmp. 135 - 138°C,

### Beispiel 32

### N-(N-(N-(N-((1,1-Dimethyl)ethoxycarbonyl)-L-alanyl)-L-alanyl)-L-prolyl)-L-valin [6α-fluor-11β,21-dihydroxy-16α-methyl-3,20-dioxo-pregna-1,4-dien-21-yl] ester (Boc-Ala-Ala-Pro-Val-O-FC)

884 mg (1.50 mmol) H-Val-O-FC x TFA (Beispiel 31), 500 mg (1.40 mmol) Boc-Ala-Ala-Pro-OH und 168 mg (1.40 mmol) N-Hydroxybenzotriazol werden in dieser Reihenfolge in 50 mL Dichlormethan gelöst und 330 µL(3.00 mmol) N-Methylmorpholin zugegeben. Dann wird eine Lösung von 289 mg (1.40 mmol) Dicyclohexylcarbodiimid in 2 mL Dichlormethan zugesetzt und 2h bei Raumtemperatur gerührt. Dann wird vom ausgefallenen Harnstoff abgesaugt, die Filtrate eingeengt und nochmals filtriert. Chromatographie dieser Rohmenge (300 g Kieselgel, Hexan/Aceton 1:1) ergibt 861 mg (76%) N-(N-(N-(N-((1,1-Dimethyl)ethoxycarbonyl)-L-alanyl)-L-alanyl)-L-prolyl)-L-valin [6α-fluor-11β,21-dihydroxy-16α-methyl-3,20-dioxo-pregna-1,4-dien-21-yl] ester. Umkristallisation aus Dichlormethan/ Diisopropylether erhält man 737 mg Reinprodukt.
Schmp. ab 136° C Zersetzung, [α]_{D} = 0° (c = 0.5% in Chloroform), HPLC: 98%. Racemtest (GC): D-Ala 3.0% D-Pro 1.9% D-Val <1%.

### Beispiel 33

### N-(N-(N-(N-(9H-Fluoren-9-yl-methoxycarbonyl)-L-alanyl)-L-alanyl)-L-prolyl)-L-valin [6α-fluor-11β,21-dihydroxy-16α-methyl-3,20-dioxo-pregna-1,4-dien-21-yl]-pregna-1,4-dien-21-yl] ester (Fmoc-Ala-Ala-Pro-Val-O-FC)

442 mg (0.75 mmol) H-Val-O-TCA x TFA (Beispiel 31), 336 mg (0.70 mmol) Fmoc-Ala-Ala-Pro-OH und 84 mg (0.70 mmol) N-Hydroxybenzotriazol werden in dieser Reihenfolge in 20 mL Dichlormethan gelöst und 155 µL(1.50 mmol) N-Methylmorpholin zugegeben. Dann wird eine Lösung von 145 mg (0.70 mmol) Dicyclohexylcarbodiimid in 2 mL Dichlormethan zugesetzt und 6h bei Raumtemperatur gerührt. Dann wird vom ausgefallenen Harnstoff abgesaugt, die Filtrate eingeengt und nochmals filtriert. Chromatographie dieser Rohmenge (300 g Kieselgel, Dichlormethan →Dichlormethan/Aceton 1:1) ergibt 298 mg ( = 44%) N-(N-(N-(N-(9H-Fluoren-9-yl-methoxycarbonyl-L-alanyl)-L-alanyl)-L-prolyl)-L-valin [6α-fluor-11β,21-dihydroxy-16α-methyl-3,20-dioxo-pregna-1,4-dien-21-yl]-pregna-1,4-dien-21-yl] ester. Umkristallisation aus Dichlormethan/Diisopropylether ergibt 158 mg reine Substanz. Dieses Material enthielt noch mehrere Verunreinigungen und wird durch HPLC (Novapak, MeCN/10mM Ammoniumhydrogencarbonat 60:40) weiter gereinigt.
Schmp. ab 158° C Zersetzung, [α]_{D} = +1° (c = 0.5% in Chloroform), HPLC: 99%. Racemtest (GC): D-Ala 1.0% D-Pro 1.4% D-Val 3.5%.

### Beispiel 34

### N-(N-(N-(L-alanyl)-L-alanyl)-L-prolyl)-L-valin [6α-fluor-11β,21-dihydroxy-16α-methyl-3,20-dioxo-pregna-1,4-dien-21-yl]-pregna-1,4-dien-21-yl]ester hydrochlorid (H-Ala-Ala-Pro-Val-O-FC x HCI)

113 mg (0.14 mmol) Boc-Ala-Ala-Pro-Val-O-FC (Beispiel 32) werden in 1 ml salzsaurem Dioxan (4N HCI in Dioxan) gelöst. Nach 6h bei Raumtemperatur wird das Lösemittel i. Vak. entfernt, der Rückstand aus Dichlormethanl/Diisopropylether umkristallisiert. Man erhält 117 mg (>100%) N-(N-(N-(L-alanyl)-L-alanyl)-L-prolyl)-L-valin [6α-fluor-11β,21-dihydroxy-16α-methyl-3,20-dioxo-pregna-1,4-dien-21-yl]-pregna-1,4-dien-21-yl] ester hydrochlorid.
HPLC: 98.3 - 99.3%, Racemtest (GC) : D-Ala 6.4% D-Pro 3.4% D-Val 1.5%.

### Beispiel 35

### N-(N-(N-(N-(Phenylcarbonyl)-L-alanyl)-L-alanyl)-L-prolyl)-L-valin [6α-fluor-11β,21-dihydroxy-16α-methyl-3,20-dioxo-pregna-1,4-dien-21-yl]-pregna-1,4 -dien-21-yl] ester (Bz-Ala-Ala-Pro-Val-O-FC)

105 mg (0.13 mmol) H-Ala-Ala-Pro-Val-O-FC x HCI (Beispiel 34) werden in 3 ml Dichlormethan und 3 ml Triethylamin gelöst, mit 23 µl (0.20 mmol) Benzoylchlorid versetzt und 8h bei Raumtemperatur gerührt. Zur Aufarbeitung wird mit 1N HCI und Wasser gewaschen, über Natriumsulfat getrocknet und i.Vak. eingeengt. Der nach Entfernung des Lösemittels i. Vak. verbleibende Rückstand wird direkt chromatographiert. Gradienten-Chromatographie an 50g Kieselgel 60 (Dichlormethan → Dichlormethan/Methanol 95:5) ergibt 96 mg (84%) N-(N-(N-(N-(Phenylcarbonyl)-L-alanyl)-L-alanyl)-L-prolyl)-L-valin [6α-fluor-11β,21-dihydroxy-16α-methyl-3,20-dioxo-pregna-1,4-dien-21-yl]-pregna-1,4-dien-21-yl] este, 75 mg (66%) aus nach Umkristallisation aus Dichlormethan/Diisopropylethert 36 mg reine Verbindung ergibt.
HPLC: 96.1 - 96.9%, Racemtest (GC): D-Ala 8.1% D-Pro <1% D-Val 1.4%.

### Beispiel 36

### (2S)-2-((1,1-Dimethylethoxycarbonyl)-amino)-3-methyl-buttersäure [11β,21-dihydroxy-3,20-dioxo-9-fluor-16β-methyl-17-valeroyloxy-pregna-1,4-dien-21-yl]ester (Boc-Val-O-BMV)

21 mmol Betamethason-17-valerat werden mit 25 mmol Boc-Valin (Boc-Val-OH), 2.5 mmol 4-Dimethylaminopyridin und 27 mmol Dicyclohexylcarbodiimidazol in Dichlormethan/Dioxan 1:1 analog Beispiel 1 umgesetzt. Chromatographie (1. Essigester/ Hexan 2:1 und 2. Essigester/Hexan 1:1) ergibt 10.1 g (73%) reines (2S)-2-((1,1-Dimethylethoxycarbonyl)-amino)-3-methyl-buttersäure [11β,21-dihydroxy-3,20-dioxo-9-fluor-16β-methyl-17-valeroyloxy-pregna-1,4-dien-21-yl] ester als Schaum.
[α]_{D} = +51° (c = 0.5% in Chloroform).

| | | | | |
|---|---|---|---|---|
| Ber. | C 65.86 | H 7.92 | N 2.08 | F 2.82 |
| Gef. | C 65.53 | H 7.72 | N 2.24 | F 2.69 |

### Beispiel 37

### (2S)-2-Amino-3-methyl-buttersäure [11β,21-dihydroxy-3,20-dioxo-9-fluor-16β-methyl-17-valeroyloxy-pregna-1,4-dien-21-yl] ester Trifluoracetat (H-Val-O-BMV x TFA)

8.5 g (13 mmol) Boc-Val-O-BMV (Beispiel 36) werden mit Trifluoressigsäure/Dichlormethan analog Beispiel 2 umgesetzt. Die Verbindung ist nicht kristallin und wird als Rohprodukt weiter verwendet. C-NMR entspricht der erwarteten Struktur.

### Beispiel 38

### N-(N-(N-(N-((1,1-Dimethyl)ethoxycarbonyl)-L-alanyl)-L-alanyl)-L-prolyl)-L-valin [9α-fluor-11β,21-dihydroxy-16β-methyl-3,20-dioxo-17-valeroyloxy-pregna-1,4-dien-21-yl] ester (Boc-Ala-Ala-Pro-Val-O-BMV)

1.04g (1.50 mmol) H-Val-O-BMV x TFA (Beispiel 37), 500 mg (1.40 mmol) Boc-Ala-Ala-Pro-OH und 168 mg (1.40 mmol) N-Hydroxybenzotriazol werden in dieser Reihenfolge in 50 mL Dichlormethan gelöst und 330 µL(3.00 mmol) N-Methylmorpholin zugegeben. Dann wird eine Lösung von 289 mg (1.40 mmol) Dicyclohexylcarbodiimid in 2 mL Dichlormethan zugesetzt und 20h bei Raumtemperatur gerührt. Dann wird vom ausgefallenen Harnstoff abgesaugt, die Filtrate eingeengt und nochmals filtriert. Chromatographie dieser Rohmenge (300 g Kieselgel, Hexan/Aceton 1:1) ergibt 806 mg (63%) N-(N-(N-(N-((1,1-Dimethyl)ethoxycarbonyl)-L-alanyl)-L-alanyl)-L-prolyl)-L-valin [9α-fluor-11β,21-dihydroxy-16β-methyl-3,20-dioxo-17-valeroyloxy-pregna-1,4-dien-21-yl] ester. Kristallisation aus Dichlormethan/ Diisopropylether 542 mg (42%).
Schmp. ab 137° C Zersetzung, [α]_{D} = 0° (c = 0.5% in Chloroform), HPLC: 98.1 - 99.2%. Racemtest (GC) : D-Ala 2.1% D-Pro 2.2% D-Val 0.6%.

### Beispiel 39

### N-(N-(N-(N-(9H-Fluoren-9-yl-methoxycarbonyl)-L-alanyl)-L-alanyl)-L-prolyl)-L-valin [9α-fluor-11β,21-dihydroxy-16β-methyl-3,20-dioxo-17-valeroyloxy-pregna-1,4-dien-21-yl] ester (Fmoc-Ala-Ala-Pro-Val-O-BMV)

689 mg (1.00 mmol) H-Val-O-BMV x TFA (Beispiel 37) , 384 mg (0.80 mmol) Fmoc-Ala-Ala-Pro-OH und 96 mg (0.80 mmol) N-Hydroxybenzotriazol werden in dieser Reihenfolge in 20 mL Dichlormethan gelöst und 155 µL(1.50 mmol) N-Methylmorpholin zugegeben. Dann wird eine Lösung von 145 mg (0.70 mmol) Dicyclohexylcarbodiimid in 2 mL Dichlormethan zugesetzt und 6h bei Raumtemperatur gerührt. Dann wird vom ausgefallenen Harnstoff abgesaugt, die Filtrate eingeengt und nochmals filtriert. Die erhaltene Lösung wird mit Dichlormethan verdünnt, mit 0.5 N NaOH und 0.5 N HCI extrahiert, über Natriumsulfat getrocknet und i. Vak. eingeengt. Chromatographie der Rohmenge (300 g Kieselgel, Dichlormethan/Aceton 1:1) ergibt 232 mg (28%) N-(N-(N-(N-(9H-Fluoren-9-yl-methoxycarbonyl)-L-alanyl)-L-alanyl)-L-prolyl)-L-valin [9α-fluor-11β,21-dihydroxy-16β-methyl-3,20-dioxo-17-valeroyloxy-pregna-1,4-dien-21-yl] ester, und nach Kristallisation aus Dichlormethan/Diisopropylether 200 mg Reinprodukt
Schmp. ab 143° C Zersetzung, [α]_{D} = -13° (c = 0.5% in Chloroform), HPLC: 95.4 - 99%. ASA: Ala 1.98 Pro 0.99 Val 1.03, Racemtest (GC): D-Ala 8.0% D-Pro 4.7% D-Val 2.6%.

### Beispiel 40

### N-(N-(N-(L-alanyl)-L-alanyl)-L-prolyl)-L-valin [9α-fluor-11β,21-dihydroxy-16β-methyl-3,20-dioxo-17-valeroyloxy-pregna-1,4-dien-21-yl] ester hydrochlorid (H-Ala-Ala-Pro-Val-O-BMV x HCI)

137 mg (0.15 mmol) Boc-Ala-Ala-Pro-Val-O-BMV (Beispiel 38) werden in 1 ml salzsaurem Dioxan (4N HCI in Dioxan) gelöst. Nach 6h bei Raumtemperatur wird das Lösemittel i. Vak. entfernt und der Rückstand aus Dichlormethanl/Diisopropylether umkristallisiert. Man erhält 114 mg (89%)N-(N-(N-(L-alanyl)-L-alanyl)-L-prolyl)-L-valin [9α-fluor-11β,21-dihydroxy-16β-methyl-3,20-dioxo-17-valeroyloxy-pregna-1,4-dien-21-yl] ester hydrochlorid.
HPLC: 97.0 - 99.2%, Racemtest (GC): D-Ala 4.9% D-Pro 3.8% D-Val 1.1%.

### Beispiel 41

### N-(N-(N-(N-(Phenylcarbonyl)-L-alanyl)-L-alanyl)-L-prolyl)-L-valin [9α-fluor-11β,21-dihydroxy-16β-methyl-3,20-dioxo-17-valeroyloxy-pregna-1,4-dien-21-yl] ester (Bz-Ala-Ala-Pro-Val-O-BMV)

102 mg (0.12 mmol) H-Ala-Ala-Pro-Val-O-BMV x HCI **40c** werden in 3 ml Dichlormethan gelöst, mit 21 µl (0.18 mmol) Benzoylchlorid und 356 µl (2.50 mmol) Diisopropylethylamin versetzt und 5h bei Raum temperatur gerührt Zur Aufarbeitung wird mit IN HCI und Wasser gewaschen, über Natriumsulfat getrocknet und i.Vak. eingeengt. Der nach Entfernung des Lösemittels i. Vak. verbleibende Rückstand wird direkt chromatographiert. Gradienten-Chromatographie an 50g Kieselgel 60 (Dichlormethan → Dichlormethan/Methanol 95:5) ergibt 79 mg (72%) N-(N-(N-(N-(Phenylcarbonyl)-L-alanyl)-L-alanyl)-L-prolyl)-L-valin [9α-fluor-11β,21-dihydroxy-16β-methyl-3,20-dioxo-17-valeroyloxy-pregna-1,4-dien-21-yl] ester, und nach Umkristallisation 56 mg (51%) kristallines Produkt aus Essigester/Hexan.
HPLC: 95%, Racemtest (GC): D-Ala 12.1% D-Pro 3.1% D-Val 1.4%.

### Beispiel 42

### (2S)-2-((1,1-Dimethylethoxycarbonyl)-amino)-3-methyl-buttersäure [11β,21-dihydroxy-3,20-dioxo-9-fluor-16α,17-[(1-methylethyliden)bis(oxy)]-pregna-1,4-dien-21-yl] ester (Boc-Val-O-TCA)

11 mmol Triamcinolon-Acetonid (TCA) werden mit 13 mmol Boc-Valin (Boc-Val-OH), 1.2 mmol DMAP und 14 mmol DCC in Dichlormethan/Dioxan 1:1 analog Beispiel 1 umgesetzt. Chromatographie (Essigester/ Hexan 2:1) ergibt 3.72 g (53%) (2S)-2-((1,1-Dimethylethoxycarbonyl)-amino)-3-methylbuttersäure [11β,21-dihydroxy-3,20-dioxo-9-fluor-16α,17-[(1-methylethyliden)bis(oxy)]-pregna-1,4-dien-21-yl] ester. Nochmalige Kristallisation aus Essigester/Hexan liefert 5.52 g (52%) Reinprodukt.
Schmp. 238 - 240° C, [α]_{D} = +75° (c = 0.5% in Chloroform).

| | | | | |
|---|---|---|---|---|
| Ber. | C 64.54 | H 7.49 | N 2.21 | F 3.00 |
| Gef. | C 64.23 | H 7.21 | N 2.35 | F 2.88 |

### Beispiel 43

### (2S)-2-Amino-3-methyl-buttersäure [11β,21-dihydroxy-3,20-dioxo-9-fluor-16α,17-[(1-methylethyliden)bis(oxy)]-pregna-1,4-dien-21-yl]ester Trifluoracetat (H-Val-O-TCA x TFA)

2.5 g (4.0 mmol) Boc-Val-O-TCA (Beispiel 42) werden mit Trifluoressigsäure/Dichlormethan analog Beispiel 2 umgesetzt. Kristallisation aus Dichlormethan/Diisopropylether ergibt 2.58 g (100%) (2S)-2-Amino-3-methyl-buttersäure [11β,21-dihydroxy-3,20-dioxo-9-fluor-16α,17-[(1 -methylethyliden) bis(oxy)]-pregna-1,4-dien-21-yl] ester Trifluoracetat.

### Beispiel 44

### N-(N-(N-(N-((1,1-Dimethyl)ethoxyearbonyl)-L-alanyl)-L-alanyl)-L-prolyl)-L-valin [11β,21-dihydroxy-3,20-dioxo-9-fluor-16α,17-[(1 -methylethyliden)bis(oxy)]-pregna-1,4-dien-21-yl]ester (Boc-Ala-Ala-Pro-Val-O-TCA)

900 mg (1.50 mmol) H-Val-O-TCA x TFA (Beispiel 43), 500 mg (1.40 mmol) Boc-Ala-Ala-Pro-OH und 168 mg (1.40 mmol) N-Hydroxybenzotriazol werden in dieser Reihenfolge in 50 mL Dichlormethan gelöst und 300 µL(3.00 mmol) N-Methylmorpholin zugegeben. Dann wird eine Lösung von 289 mg (1.40 mmol) Dicyclohexylcarbodiimid in 2 mL Dichlormethan zugesetzt und 2h bei Raumtemperatur gerührt. Dann wird vom ausgefallenen Harnstoff abgesaugt, die Filtrate eingeengt und nochmals filtriert. Chromatographie dieser Rohmenge (300 g Kieselgel, Hexan/Aceton 1:1) ergibt 935 mg (71%) N-(N-(N-(N-((1,1-Dimethyl)ethoxycarbonyl)-L-alany)-L-alany)-L-prolyl)-L-valin [11β,21-dihydroxy-3,20-dioxo-9-fluor-16α,17-[(1-methylethyliden)bis(oxy)]-pregna-1,4-dien-21-yl] ester. Nochmalige Kristallisation aus Dichlormethan/ Diisopropylether liefert 817 mg Reinprodukt.
Schmp. ab 151°C Zersetzung, [α]_{D} = -1° (c = 0.26% in Chloroform), HPLC: 93 - 95%. Racemtest (GC): D-Ala 3.0% D-Pro 1.9% D-Val <1%.

### Beispiel 45

### N-(N-(N-(N-(9H-Fluoren-9-yl-methoxycarbonyl)-L-alanyl)-L-alanyl)-L-prolyl)-L-valin [11β,21-dihydroxy-3,20-dioxo-9-fluor-16α,17-[(1-methylethyliden)bis (oxy)]-pregna-1,4-dien-21-yl] ester (Fmoc-Ala-Ala-Pro-Val-O-TCA)

485 mg (0.75 mmol) H-Val-O-TCA x TFA (Beispiel 43), 336 mg (0.70 mmol) Fmoc-Ala-Ala-Pro-OH und 84 mg (0.70 mmol) N-Hydroxybenzotriazol werden in dieser Reihenfolge in 20 mL Dichlormethan gelöst und 155 µL(1.50 mmol) N-Methylmorpholin zugegeben. Dann wird eine Lösung von 145 mg (0.70 mmol) Dicyclohexylcarbodiimid in 2 mL Dichlormethan zugesetzt und 12h bei Raumtemperatur gerührt. Dann wird vom ausgefallenen Harnstoff abgesaugt, die Filtrate eingeengt und nochmals filtriert. Chromatographie dieser Rohmenge (300 g Kieselgel, Dichlormethan →Dichlormethan/Aceton 1:1) ergibt 215 mg (30%) N-(N-(N-(N-(9H-Fluoren-9-yl-methoxycarbonyl)-L-alanyl)-L-alanyl)-L-prolyl)-L-valin [11β,21-dihydroxy-3,20-dioxo-9-fluor-16α,17-[(1-methylethyliden)bis(oxy)]-pregna-1,4-dien-21-yl] ester, welches nach Kristallisation aus Dichlormethan/Diisopropylether und Essigester/Hexan kein reines Produkt ergibt. Auch eine zweite Chromatographie (100 g Kieselgel, Hexan/Aceton 1:1) bleibt ebenfalls erfolglos. Die verbleibenden 50 mg werden durch präparative HPLC (Novapak, MeCN/10mM Ammoniumhydrogencarbonat 60:40) gereinigt.
HPLC: 99.3 - 99.6%. Racemtest (GC): D-Ala 1.0% D-Pro 0.8% D-Val 3.2%.

### Beispiel 46

### N-(N-(N-(L-alanyl)-L-alanyl)-L-prolyl)-L-valin [11ß,21-dihydroxy-3,20-dioxo-9-fluor-16α,17-[(1-methylethyliden)bis(oxy)]-pregna-1,4-dien-21-yl] ester hydrochlorid (H-Ala-Ala-Pro-Val-O-TCA x HCI)

131 mg (0.15 mmol) Boc-Ala-Ala-Pro-Val-O-TCA (Beispiel 44) werden in 1 ml salzsaurem Dioxan (4N HCI in Dioxan) gelöst. Nach 6h bei Raumtemperatur wird das Lösemittel i. Vak. entfernt und der Rückstand aus Dichlormethanl/Diisopropylether umkristallisiert. Man erhält 115 mg (95%) N-(N-(N-(L-alanyl)-L-alanyl)-L-prolyl)-L-valin[11β,21-dihydroxy-3,20-dioxo-9-fluor-16α,17-[(1-methylethyliden)bis(oxy)]-pregna-1,4-dien-21-yl] ester hydrochlorid.
Racemtest (GC): D-Ala 2.1% D-Pro <1% D-Val 0.8%.

### Beispiel 47

### N-(N-(N-(N-(Phenylcarbonyl)-L-alanyl)-L-alanyl)-L-prolyl)-L-valin [11β,21-dihydroxy-3,20-dioxo-9-fluor-16α,17-[(1-methylethyliden)bis(oxy)]-pregna-1,4 -dien-21-yl] ester (Bz-Ala-Ala-Pro-Val-O-TCA)

105 mg (0.13 mmol) H-Ala-Ala-Pro-Val-O-TCA x HCI (Beispiel 46) werden in 3 ml Dichlormethan und 3 ml Triethylamin gelöst, mit 23 µl (0.20 mmol) Benzoylchlorid versetzt und 8h bei Raumtemperatur gerührt. Zur Aufarbeitung wird mit 1N HCI und Wasser gewaschen, über Natriumsulfat getrocknet und i.Vak. eingeengt. Der nach Entfernung des Lösemittels i. Vak. verbleibende Rückstand wird direkt chromatographiert. Gradienten-Chromatographie an 50g Kieselgel 60 (Dichlormethan → Dichlormethan/Methanol 95:5) ergibt 85 mg (76%) N-(N-(N-(N-(Phenylcarbonyl)-L-alanyl)-L-alanyl)-L-prolyl)-L-valin [11β,21-dihydroxy-3,20-dioxo-9-fluor-16α,17-[(1-methylethyliden)bis(oxy)]-pregna-1,4-dien-21-yl] ester, und nach Umkristallisation 56 mg (49%) kristallines Reinprodukt aus Essigester/Hexan.

### Biologische Daten

### 1. Bindung an den Glucocorticoid-Rezeptor

Die Bindung der erfindungsgemäßen Substanzen an den Ratten-Glucocorticoid-Rezeptor wird durch kompetitive Verdrängung tritiierten Dexamethasons aus der Rezeptorbindung ermittelt. Unterschiedliche Konzentrationen der Glucocorticoid-Ester werden für 2 Stunden bei 4°C in Anwesenheit einer konstanten Konzentration von Tritium-markiertem Dexamethason mit Rattenthymus-Zytosol inkubiert. Nach der Inkubationszeit werden die nicht proteingebundenen Steroide an Aktivkohle adsorbiert und die Aktivkohle abzentrifugiert. Die Radioaktivität im Überstand dient als Maß für die Menge des Rezeptor-gebundenen Dexamethasons.
Aus den Messungen werden die Konzentrationen des jeweiligen Oorticoid-Derivats errechnet, die notwendig sind, um 50 % des markierten Dexamethasons aus der Rezeptorbindung zu verdrängen. Der Kompetitionsfaktor (KF) ist der Quotient aus dieser errechneten Konzentrationen für die Testsubstanz und der für Dexamethason. Je besser eine Verbindung an den Rezeptor bindet, desto niedriger ist ihr KF.
Wie aus Tabelle 1 ersichtlich, binden die erfindungsgemäßen Glucocorticoid-Oligopeptid-Ester deutlich schlechter als die unveresterten Glucocorticoid-Grundkörper an den Glucocorticoid-Rezeptor des Rattenthymus. Insofern erfüllen sie die Bedingung eines Prodrugs.

**Tabelle 1:**

| Kompetitionsfaktoren am cytosolischen Rattenthymus-Glucocorticoid-Rezeptor | | |
|---|---|---|
| Corticoid | Beispiel | Kompetitionsfaktor [KF] |
| 6α-Methylprednisolon-17-propionat | | 0.8 |
| Boc-Val-O-MPP | 1 | 17 |
| Boc-Ala-Ala-Pro-Val-O-MPP | 3 | 5.6 |
| Fmoc-Ala-Ala-Pro-Val-O-MPP | 4 | 100 |
| Ac-Ala-Ala-Pro-Val-O-MPP | 7 | 20 |
| Bz-Ala-Ala-Pro-Val-O-MPP | 8 | 24 |
| Valeroyl-Ala-Ala-Pro-Val-O-MPP | 9 | 7 |
| 6α-Methylprednisolon | | 0.9 |
| Boc-Val-O-MP | 19 | 144 |
| Boc-Ala-Ala-Pro-Val-O-MP | 21 | 56 |
| Fmoc-Ala-Ala-Pro-Val-O-MP | 22 | 220 |
| Hydrocortison | | 4 |
| Boc-Val-O-HC | 27 | >1000 |
| Fmoc-Ala-Ala-Pro-Val-O-HC | 29 | k.k. |
| Boc-Ala-Ala-Pro-Val-O-FC | 32 | 5 |
| Boc-Ala-Ala-Pro-Val-O-TCA | 44 | 24 |
| Bz-Ala-Ala-Pro-Val-O-TCA | 47 | 10 |
| (k.k. = keine Kompetition) | | |

### 2. Spaltung im Homogenat normaler Rattenhaut

Das Homogenat von Haut männlicher Ratten (0.1 mol/l Phosphatpuffer, pH = 7.4) wird 20 Minuten bei 10.000 g zentrifugiert Der Überstand wird in Phosphatpuffer auf eine Proteinkonzentration von 3 mg/ml eingestellt. Zu 1 ml dieses Homogenats werden die Testsubstanzen in 20 µl Ethanol zugegeben. Die Endkonzentration der Corticoid-Derivate liegt bei 100 µmol/l. Die Inkubation erfolgt für unterschiedliche Zeiten bei 37 °C. Am Ende der Inkubation werden die Proben 3mal mit 3 ml Chloroform extrahiert. Die vereinigten Extrakte werden unter Stickstoff zur Trockne eingedampft und mit 100 µl Ethanol aufgenommen. Die ethanolischen Lösungen werden mit dem gleichen Volumen Wasser versetzt und die Verseifungsprodukte mittels HPLC (RP 18, Acetonitril/Wasser-Gradient, Detektion 240 nm) aufgetrennt. Die relative Hydrolyserate ist in Prozent der Hydrolyserate des 6α-Methylprednisolon-17-Propionat-21-Acetats angegeben.
Die Oligopeptid-Ester erweisen sich als weniger hydrolyseempfindlich als einfache Carbonsäure-Ester (Tabelle 2).

**Tabelle 2:**

| Unspezifische Spaltung der Corticoid-21-Oligopeptid-Ester durch Esterasen der Rattenhaut | | |
|---|---|---|
| Corticoid | Beispiel | Relative Hydrolyserate [%] |
| 6α-Methylprednisolon-17-propionat-21-acetat | | 100 |
| Boc-Ala-Ala-Pro-Val-O-MPP | 3 | 50 |
| Fmoc-Ala-Ala-Pro-Val-O-MPP | 4 | 4 |
| DPAc-Ala-Ala-Pro-Val-O-MPP | 10 | 6 |
| DPC-Ala-Ala-Pro-Val-O-MPP | 11 | 14 |
| Naphthoyl-Ala-Ala-Pro-Val-O-MPP | 12 | 4 |
| Cinnamoyl-Ala-Ala-Pro-Val-O-MPP | 13 | 5 |
| Cbs-Ala-Ala-Pro-Val-O-MPP | 14 | 6 |
| Cbp-Ala-Ala-Pro-Val-O-MPP | 16 | 3 |
| Pht-Gly-Ala-Ala-Pro-Val-O-MPP | 17 | 49 |
| Fmoc-Ala-Ala-Pro-Val-O-MP | 22 | 6 |
| Boc-Ala-Ala-Pro-Val-O-BMV | 38 | 0 |
| Fmoc-Ala-Ala-Pro-Val-O-BMV | 39 | 0 |

### 3. Spaltung der Glucocorticoid-21-Oligopeptid-Ester durch Leukozyten-Elastase

Polymorphkemige Granulozyten werden aus Spenderblut isoliert. Die Zellen werden in einer Dichte von 10⁶ Zellen pro ml lysiert. Je 1 ml des Lysats wird mit 20 µl ethanolischer Corticoidlösung (5 mmol/l) versetzt. Nach 1 stündiger Inkubation bei 37 °C werden die Proben dreimal mit 3 ml Chloroform extrahiert und wie oben beschrieben zur HPLC-Analyse aufgearbeitet. Die Spaltung der erfindungsgemäßen Prodrugs ist unter diesen Versuchsbedingungen durch Zugibte des spezifischen Elastase-Inhibitors MeO-Succinyl-Ala-Ala-Pro-Val-Chlormethylketon zu 90% hemmbar. Außerdem werden die Verbindungen auch durch reine humane Sputum-Elastase (Elastin Products) hydrolysiert. Diese Befunde legen eine spezifische Spaltung der Prodrugs durch Elastase nahe.

**Tabelle 3:**

| Spaltung von Glucocorticoid-21-Ester im Lysat humaner neutrophiler Granulozyten | | |
|---|---|---|
| Corticoid | Beispiel | Relative Hydrolyserate [%] |
| Boc-Ala-Ala-Pro-Val-O-MPP | 3 | 160 - 190 |
| Fmoc-Ala-Ala-Pro-Val-O-MPP | 4 | 100 |
| Z-Ala-Ala-Pro-Val-O-MPP | 5 | 160 - 230 |
| Fmoc-Aib-Ala-Ala-Pro-Val-O-MPP | 18 | 130 |
| Fmoc-Ala-Ala-Pro-Val-O-MP | 22 | 370 |
| Boc-Ala-Ala-Pro-Val-O-FC | 32 | 380 |
| Fmoc-Ala-Ala-Pro-Val-O-BMV | 39 | 42 |
| Fmoc-Ala-Ala-Pro-Val-O-MPP wird als Referenz verwendet. | | |

### 4. Lokale antiinflammatorische Wirksamkeit

Die antiinflammatorische Aktivität der Corticoid-21-Oligopeptid-Ester wird in einem modifizierten Rattenohr-Test nach Tonelli durchgeführt. Die Modifikationen beruhen auf Veränderungen der Reizlösung (4 % Crotonöl, 10 % DMSO in Ethanol), der Applikationsart (Auftragen der Lösung mit Pipette) und der Auswertung des Versuchs 16 Stunden nach Applikation der Reizlösung. Zu diesem Zeitpunkt läßt sich sowohl die Hemmung des Ödems durch Messung des Ohrgewichtes als auch die Hemmung des granulozytären Infiltrates durch Messung der Granulozytenmarker Myeloperoxidase und Leukozyten-Elastase im Ohrhomogenat bestimmen. Die Gruppengröße betrug jeweils 6 Tiere. Wie aus Tabelle 4 ersichtlich, ist die antientzündliche Aktivität der Corticoid-21-Oligopeptid-Ester nach lokaler Applikation der Aktivität der homologen Corticoid-21-Acetate vergleichbar.

**Tabelle 4:**

| Antientzündliche Wirksamkeit von Glucocorticoid-21-Estern nach lokaler Applikation (Rattenohr-Test) | | | |
|---|---|---|---|
| Corticoid | Konzentration [% w/v] | Ödem [% Hemmung] | Infiltration [% Hemmung] |
| 6α-Methylprednisolon- 17-propionat-21-acetat | 0.3 | 67* | 78* |
| | 0.03 | 62* | 45* |
| | 0.003 | 44* | 12 |
| Boc-Ala-Ala-Pro-Val-O- MPP (Beispiel 3) | 0.3 | 81* | 77* |
| | 0.03 | 51* | 43* |
| | 0.003 | 17 | 11 |
| Fmoc-Ala-Ala-Pro-Val- O-MPP (Beispiel 4) | 0.3 | 78* | 66* |
| | 0.03 | 48* | 42* |
| | 0.003 | 30* | 16* |

| | | | |
|---|---|---|---|
| * Signifikante Hemmung (p < 0.05) gegenüber Positivkontrolle (Applikation von Crotonöl ohne Corticoid) | | | |

## Patentansprüche

1. Glucocorticoide der allgemeinen Formel **I**
R-Val-O-GC (**I**),
worin
O-GC der Rest eines antiinflammatorisch wirksamen 21-Hydroxycorticoids ist,
Val einen in der 21-Position des Corticoids befindlichen Valinrest darstellt und
R ein Wasserstoffatom oder einen gegebenenfalls durch Hydroxygruppen, Aminogruppen, Oxogruppen und/oder Halogenatome substituierten und/oder durch Sauerstoffatome, SO₂-Gruppen und/oder NH-Gruppen unterbrochener Kohlenwasserstoffrest mit bis zu 32 Kohlenstoffatomen bedeutet
und deren Salze.

2. Glucocorticoide der allgemeinen Formel **II.** 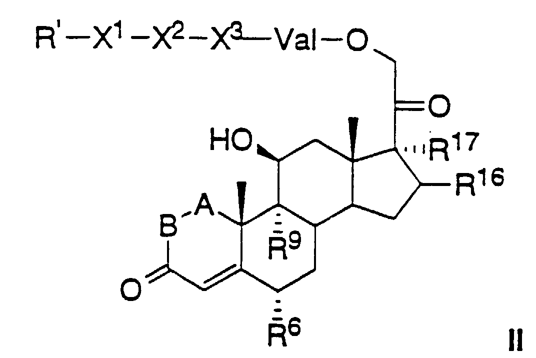
worin
R' = H, CH=O (C=O)R", (C=O)OR" oder SO₂R"
X¹ - X³ = unabhängig voneinander Alanin, Prolin oder Valin,
A-B = CH₂-CH₂ oder CH=CH
R⁶= H,F,Cl,Me,
R⁹ = H, F, Cl,
R¹⁶ = H,Me,OH,
R¹⁷ = H, OH, O(C=O)R'" oder
R¹⁶ R¹⁷ = Alkylidendioxy ist, in denen
R" ein C₁-C₁₈ enthaltendes Kohlenwasserstoff-Radikal und
R"' ein C₁-C₁₀ Alkyl (gerad- oder verzweigt-kettig) Aryl, Alkylaryl oder C₁-C₃ Alkoxy Radikal darstellen und
der Alkylidenrest sich von einem 1-6 C-Atome enthaltenden aliphatischen Aldehyd, einem 3-6 C-Atome enthaltendem Keton oder 5-6 C-Atome enthaltendem cyclischen Keton oder Benzaldehyd ableitet und deren Salze.

3. Glucocorticoide der allgemeinen Formel I und II, dadurch gekennzeichnet, daß X¹ und X² Alanin sowie X³ Prolin oder Valin bedeutet.

4. Pharmazeutische Präparate, gekennzeichnet durch einen Gehalt an Glucocorticoiden gemäß Anspruch 1 oder 2.

## Claims

1. Glucocorticoids of the general formula I
R-Val-O-GC (I),
wherein
O-GC is the portion of a 21-hydroxycorticoid, which 21-hydroxycorticoid has antiinflammatory activity,
Val represents a valine residue located in the 21-position of the corticoid and
R represents a hydrogen atom or a hydrocarbon radical having up to 32 carbon atoms optionally substituted by hydroxy groups, amino groups, oxo groups and/or by halogen atoms and/or interrupted by oxygen atoms, SO₂ groups and/or by NH groups
and salts thereof.

2. Glucocorticoids of the general formula II 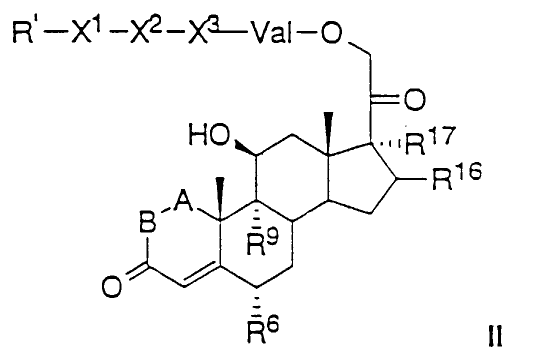 wherein
R' represents H, CH=O, (C=O)R", (C=O)OR" or SO₂R",
X¹-X³ represent, each independently of the others, alanine, proline or valine,
A-B represents CH2-CH2 or CH=CH,
R⁶ represents H, F, Cl, Me,
R⁹ represents H, F, Cl,
R¹⁶ represents H, Me, OH,
R¹⁷ represents H, OH, O(C=O)R"' or
R¹⁶, R¹⁷ represent alkylidenedioxy, wherein
R" represents a C₁-C₁₈ hydrocarbon radical and
R"' represents a C₁-C₁₀alkyl (straight-chained or branched), aryl, alkylaryl or C₁-C₃-alkoxy radical and
the alkylidene group is derived from an aliphatic aldehyde containing from 1 to 6 carbon atoms, from a ketone containing from 3 to 6 carbon atoms or from cyclic ketone containing 5 or 6 carbon atoms or from benzaldehyde,
and salts thereof.

3. Glucocorticoids of the general formula I or II, characterised in that X¹ and X² represent alanine and X³ represents proline or valine.

4. Pharmaceutical compositions, characterised in that they comprise glucocorticoids according to claim 1 or claim 2.

## Revendications

1. Glucocorticoïdes de formule générale I
R-Val-O-GC (I)
dans laquelle
O-GC est le reste d'un 21-hydroxycorticoïde à activité anti-inflammatoire,
Val représente un reste valine se trouvant en position 21 du corticoïde, et
R est un atome d'hydrogène ou un reste hydrocarboné ayant jusqu'à 32 atomes de carbone, éventuellement substitué par des groupes hydroxy, des groupes amino, des groupes oxo et/ou des atomes d'halogène et/ou interrompu par des atomes d'oxygène, des groupes SO₂ et/ou des groupes NH,
et leurs sels.

2. Glucocorticoïdes de formule générale II 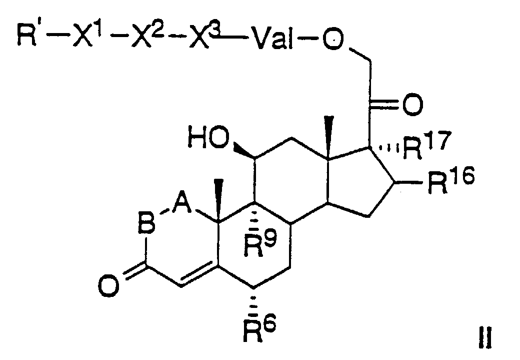 dans laquelle
R' = H, CH=O, (C=O)R", (C=O)OR" ou SO₂R",
X¹ à X³ sont, indépendamment les uns des autres, l'alanine, la proline ou la valine,
A-B = CH₂-CH₂ ou CH=CH,
R⁶ = H, F, CI, Me,
R⁹ = H, F, CI,
R¹⁶ = H, Me, OH,
R¹⁷ = H, OH, O(C=O)R"',
R¹⁶, R¹⁷ = alkylidènedioxy, où
R" est un radical hydrocarboné en C₁-C₁₈ et
R"' représente un radical alkyle en C₁-C₁₀ (linéaire ou ramifié), aryle, alkylaryle ou alcoxy en C₁-C₃, et
le reste alkylidène dérive d'un aldéhyde aliphatique de 1 à 6 atomes de carbone, d'une cétone de 3 à 6 atomes de carbone ou d'une cétone cyclique de 5 à 6 atomes de carbone ou du benzaldéhyde,
et leurs sels.

3. Glucocorticoïdes de formule générale I et II, caractérisés en ce que X¹ et X² représentent l'alanine et X³ représente la proline ou la valine.

4. Préparations pharmaceutiques caractérisées en ce qu'elles contiennent des glucocorticoïdes selon la revendication 1 ou 2.
